(19) Europäisches Patentamt
European Patent Office
Office européen des brevets

(11) **EP 4 691 476 A1**

(12) **EUROPEAN PATENT APPLICATION**
published in accordance with Art. 153(4) EPC

(43) Date of publication:
**11.02.2026 Bulletin 2026/07**

(21) Application number: **24779783.0**

(22) Date of filing: **19.03.2024**

(51) International Patent Classification (IPC):
***A61K 31/7088*** *(2006.01)* ***A61K 9/51*** *(2006.01)*
***A61K 47/12*** *(2006.01)* ***A61K 47/18*** *(2017.01)*
***A61K 47/24*** *(2006.01)* ***A61K 47/28*** *(2006.01)*
***A61K 48/00*** *(2006.01)*

(52) Cooperative Patent Classification (CPC):
**A61K 9/51; A61K 31/7088; A61K 47/12; A61K 47/18; A61K 47/24; A61K 47/28; A61K 48/00**

(86) International application number:
**PCT/JP2024/010857**

(87) International publication number:
**WO 2024/203660 (03.10.2024 Gazette 2024/40)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **29.03.2023 JP 2023053865**

(71) Applicants:
- **Tohoku University**
  **Sendai-shi, Miyagi 980-8577 (JP)**
- **National University Corporation Chiba University**
  **Chiba-shi, Chiba 263-8522 (JP)**
- **NOF Corporation**
  **Shibuya-ku**
  **Tokyo 150-6019 (JP)**

(72) Inventors:
- **AKITA, Hidetaka**
  **Sendai-shi, Miyagi 980-8577 (JP)**
- **SAKURAI, Yu**
  **Sendai-shi, Miyagi 980-8577 (JP)**
- **TANAKA, Hiroki**
  **Chiba-shi, Chiba 260-8675 (JP)**
- **TANEICHI, Sakura**
  **Kawasaki-shi, Kanagawa 210-0865 (JP)**
- **ITO, Kasumi**
  **Kawasaki-shi, Kanagawa 210-0865 (JP)**
- **TANGE, Kota**
  **Kawasaki-shi, Kanagawa 210-0865 (JP)**
- **NAKAI, Yuta**
  **Kawasaki-shi, Kanagawa 210-0865 (JP)**

(74) Representative: **Weickmann & Weickmann PartmbB**
**Postfach 860 820**
**81635 München (DE)**

(54) **METHOD FOR PRODUCING NUCLEIC ACID-ENCAPSULATED LIPID NANOPARTICLES**

(57) The present invention provides a method for producing nucleic acid-encapsulating lipid nanoparticles, including the following steps (a) and (b):
step (a) of mixing an alcohol solution containing an ionic lipid having a tertiary amino group, a sterol, and a PEG lipid with a citrate buffer having pH 3 to 6.5 in which nucleic acid is dispersed to prepare a suspension of nucleic acid-encapsulating lipid nanoparticles; and
step (b) of exchanging a dispersion medium of the aforementioned suspension for a Tris buffer having pH 5.2 to 9.0 by concentrating the suspension of nucleic acid-encapsulating lipid nanoparticles by ultrafiltration and diluting same with the aforementioned Tris buffer.

EP 4 691 476 A1

## Description

[Technical Field]

The present invention relates to a method for producing nucleic acid-encapsulating lipid nanoparticles, a method for introducing a nucleic acid into cells, and a method for producing a pharmaceutical product composition.

[Background Art]

For practicalization of nucleic acid therapy using oligonucleic acids such as siRNA, and gene therapy using mRNA, pDNA, and the like, an effective and safe nucleic acid delivery carrier is demanded. While virus vectors are nucleic acid delivery carriers with good expression efficiency, the development of non-viral nucleic acid delivery carriers that can be used more safely is ongoing.

Since cationic liposomes using cationic lipids with quaternary amine portion are positively charged, they can form a complex (lipoplex) by electrostatic interaction with negatively-charged nucleic acids, and can deliver nucleic acids into cells (see, for example, Patent Literatures 1 and 2).

However, it is difficult to control the particle size of lipoplex produced by such method, and cytotoxicity derived from positively-charged cationic lipids becomes a problem.

Therefore, lipid nanoparticles (LNP) using ionic lipids having a tertiary amino group, which is positively charged under acidic conditions and has no electric charge under near neutral conditions, in the molecule were developed, and have become non-viral nucleic acid delivery carriers most generally used at present (see, for example, Non Patent Literature 1).

As lipid nanoparticles using an ionic lipid having a tertiary amino group in the molecule, an example also exists in which a degradable group is added to the ionic lipid (see, for example, Patent Literature 3).

As described, various non-viral carriers have been developed. However, since nucleic acids are generally unstable compounds, there are still problems with the storage stability of nucleic acid-encapsulating lipid nanoparticles.

[Citation List]

[Patent Literature]

[Patent Literature 1]
JP 2008-5801 A
[Patent Literature 2]
JP 2001-2565 A
[Patent Literature 3]
WO 2013/073480
[Patent Literature 4]
WO 2019/188867

[Non Patent Literature]

[Non Patent Literature 1]
Gene Therapy (1999) 6. 271-281

[Summary of Invention]

[Technical Problem]

A known method for producing nucleic acid-encapsulating lipid nanoparticles includes mixing an acidic buffer in which nucleic acid is dispersed with a lipid solution dissolved in alcohol, and then exchanging the dispersion medium of the obtained suspension for a different buffer. For example, Patent Literature 4 describes an example in which nucleic acid-encapsulating lipid nanoparticles were preparing by using a malic acid buffer with pH 3.0 as the acidic buffer and exchanging the dispersion medium of the obtained suspension for PBS with pH 7.4. However, there is no mention of the influence of each buffer on the property of the nucleic acid-encapsulating lipid nanoparticles.

As a method for improving the stability of nucleic acid-encapsulating lipid nanoparticles, attempts have been

made to add a cryoprotectant to nucleic acid-encapsulating lipid nanoparticles and cryopreserve them, or lyophilize nucleic acid-encapsulating lipid nanoparticles and add water at the time of use to reconstitute a suspension of the nucleic acid-encapsulating lipid nanoparticles. While these methods are useful for improving the stability of nucleic acid-encapsulating lipid nanoparticles, there is room for improvement in terms of simplicity.

In view of the above-mentioned problems, the present invention aims to provide a method for producing nucleic acid-encapsulating lipid nanoparticles, which can improve storage stability of a suspension of nucleic acid-encapsulating lipid nanoparticles and which could not be achieved by the prior art.

[Solution to Problem]

The present inventors have made extensive efforts in view of the above-mentioned problem and found that the buffer used in lipid nanoparticle preparation steps affects the nucleic acid delivery efficiency of lipid nanoparticles and the storage stability of the suspension thereof. In particular, they have found that, by replacing the dispersion medium of a suspension obtained using a citrate buffer as the acidic buffer with a Tris buffer, the nucleic acid delivery efficiency of nucleic acid-encapsulating lipid nanoparticles and the storage stability of the suspension can be enhanced, and completed the present invention. Based on this finding, the present invention provides the following.

[1] A method for producing nucleic acid-encapsulating lipid nanoparticles, comprising the following steps (a) and (b):

step (a) of mixing an alcohol solution containing an ionic lipid having a tertiary amino group, a sterol, and a PEG lipid with a citrate buffer having pH 3 to 6.5 in which nucleic acid is dispersed to prepare a suspension of nucleic acid-encapsulating lipid nanoparticles; and

step (b) of exchanging a dispersion medium of the aforementioned suspension for a Tris buffer having pH 5.2 to 9.0 by concentrating the suspension of nucleic acid-encapsulating lipid nanoparticles by ultrafiltration and diluting same with the aforementioned Tris buffer.

[2] The method of the aforementioned [1], wherein a total concentration of citric acid and a salt thereof in the citrate buffer used in step (a) is 10 to 100 mM, and a total concentration of trishydroxymethylaminomethane and a salt thereof in the Tris buffer used in step (b) is 10 to 200 mM.

[3] The method of the aforementioned [1] or [2], wherein the alcohol solution used in step (a) further comprises a phospholipid.

[4] The method of any one of the aforementioned [1] to [3], wherein the ionic lipid is a compound represented by the formula (1):

[Chem. 1]

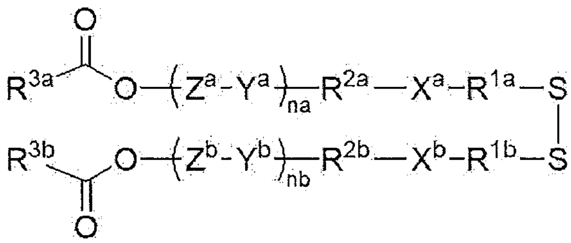

(1)

(in the formula (1),

$R^{1a}$ and $R^{1b}$ are each independently an alkylene group having 1 to 6 carbon atoms,

$X^a$ and $X^b$ are each independently an acyclic alkyl tertiary amino group having 1 to 6 carbon atoms and 1 tertiary amino group, or a cyclic alkylene tertiary amino group having 2 to 5 carbon atoms and 1 or 2 tertiary amino groups,

$R^{2a}$ and $R^{2b}$ are each independently an alkylene group having 1 to 8 carbon atoms or an oxydialkylene group having 2 to 8 carbon atoms,

$Y^a$ and $Y^b$ are each independently an ester bond, an amide bond, a carbamate bond, an ether bond, or a urea bond,

$Z^a$ and $Z^b$ are each independently a divalent group derived from an aromatic compound having 3 to 16 carbon atoms and at least one aromatic ring, and optionally having a hetero atom,

na and nb are each independently 0 or 1,

$R^{3a}$ and $R^{3b}$ are each independently a residue derived from a reaction product of a liposoluble vitamin having a hydroxyl group, and succinic anhydride or glutaric anhydride, a residue derived from a reaction product of a sterol derivative having a hydroxyl group, and succinic anhydride or glutaric anhydride, an aliphatic hydrocarbon group having 1 to 40 carbon atoms, an alkyl group having a cyclopropane ring and having 3 to 40 carbon atoms, or a group represented by the formula (3c):

$$R^{3c}\text{-O-CO-}(CH_2)_a\text{-}* \qquad (3c)$$

(in the formula (3c),
* is a bonding position,
$R^{3c}$ is an aliphatic hydrocarbon group having 2 to 20 carbon atoms, and
a is an integer of 2 to 10)).

[5] The method of any one of the aforementioned [1] to [4], wherein a molar ratio of the total amino groups of the ionic lipid to the phosphate groups of the nucleic acid (total amino groups of ionic lipid/phosphate groups of nucleic acid) used in step (a) is not less than 7.
[6] A method for introducing a nucleic acid into a cell, comprising contacting a nucleic acid-encapsulating lipid nanoparticle produced by the method of any one of the aforementioned [1] to [5] with the aforementioned cell in vitro.
[7] A method for introducing a nucleic acid into a target cell of a living organism, comprising administering nucleic acid-encapsulating lipid nanoparticles produced by the method of any one of the aforementioned [1] to [5] to the aforementioned living organism.
[8] A method for producing a pharmaceutical composition, comprising the method of any one of the aforementioned [1] to [5].

[Advantageous Effects of Invention]

The production method of the present invention can produce nucleic acid-encapsulating lipid nanoparticles that have a higher nucleic acid delivery efficiency immediately after production, compared to conventional techniques. Furthermore, a suspension of nucleic acid-encapsulating lipid nanoparticles obtained by the production method of the present invention is superior in storage stability. Since the nucleic acid-encapsulating lipid nanoparticles obtained using the production method of the present invention have high nucleic acid delivery efficiency compared to conventional techniques, they are advantageous for gene transfer in cells and living organisms, and are particularly useful as pharmaceutical compositions because of the high storage stability of the suspension thereof.

[Brief Description of Drawings]

[Fig. 1]
Fig. 1 is a graph showing the results of the nucleic acid introduction efficiency of the nucleic acid-encapsulating lipid nanoparticles obtained in Example 1 and Comparative Examples 1 to 6.
[Fig. 2]
Fig. 2 is a graph showing the change over time in the nucleic acid introduction efficiency of the nucleic acid-encapsulating lipid nanoparticles obtained in Example 1, Comparative Example 1, and Comparative Example 6.
[Fig. 3]
Fig. 3 is a graph showing the results of the nucleic acid introduction efficiency of the nucleic acid-encapsulating lipid nanoparticles obtained in Example 2 by using citrate buffer with different pHs.
[Fig. 4]
Fig. 4 is a graph showing the results of the nucleic acid introduction efficiency of the nucleic acid-encapsulating lipid nanoparticles obtained in Example 3 under conditions of different molar ratio of total amino groups in ionic lipid to phosphate groups in nucleic acid (total amino groups in ionic lipid/phosphate groups in nucleic acid).
[Fig. 5]
Fig. 5 is a graph showing the results of nucleic acid introduction efficiency of nucleic acid-encapsulating lipid nanoparticles obtained in Example 4 and Comparative Example 7.
[Fig. 6]
Fig. 6 is a graph showing the results of nucleic acid introduction efficiency of nucleic acid-encapsulating lipid nanoparticles obtained in Example 5 and Comparative Example 8.
[Fig. 7]

Fig. 7 is a graph showing the change over time in the nucleic acid introduction efficiency in vivo of the nucleic acid-encapsulating lipid nanoparticles obtained in Example 1, Comparative Example 1, and Comparative Example 6.
[Fig. 8]
Fig. 8 is a graph showing the results of the nucleic acid introduction efficiency of the nucleic acid-encapsulating lipid nanoparticles obtained in Example 6 and Comparative Example 9.

[Description of Embodiments]

While the embodiments of the present invention are explained in the following, the present invention is not limited thereto.
The production method of the nucleic acid-encapsulating lipid nanoparticles of the present invention includes the following steps (a) and (b):

step (a) of mixing an alcohol solution containing an ionic lipid having an amino group, a sterol, and a PEG lipid with a citrate buffer having pH 3 to 6.5 in which nucleic acid is dispersed to prepare a suspension of lipid nanoparticles; and
step (b) of exchanging the dispersion medium of the aforementioned suspension for a Tris buffer having pH 5.2 to 9.0 by concentrating the suspension of lipid nanoparticles by ultrafiltration and diluting same with the aforementioned Tris buffer.

According to the present invention, a suspension of nucleic acid-encapsulating lipid nanoparticles, which is superior in storage stability, can be produced. In other words, the method of the present invention is a production method of a suspension of nucleic acid-encapsulating lipid nanoparticles which is superior in storage stability.
In the present specification, the "nucleic acid-encapsulating lipid nanoparticle" means a lipid nanoparticle that contains a nucleic acid inside.
In the present specification, the "lipid nanoparticle" means a particle having a membrane structure wherein the hydrophilic groups of amphiphilic lipid are arranged in the interface, facing the aqueous phase side.
In the present specification, the "amphiphilic lipid" means a lipid having both a hydrophilic group showing hydrophilicity, and a hydrophobic group showing hydrophobicity. Examples of the amphiphilic lipid include ionic lipid, phospholipid, PEG lipid, and the like. Examples of the lipid include ionic lipid, sterol, PEG lipid, and phospholipid.

Step (a)

Ionic lipid having tertiary amino group

The alcohol solution used in step (a) contains an ionic lipid having a tertiary amino group (hereinafter sometimes to be abbreviated as "ionic lipid"). Only one type of ionic lipid may be used, or two or more types thereof may be used in combination. The ionic lipid that can be used in the present invention is any ionic lipid composed of a tertiary amino group and a hydrophobic group and capable of forming lipid nanoparticles.
Examples of the ionic lipid include 1,2-dioleoyloxy-3-dimethylaminopropane (DODAP), 1,2-dioleyloxy-3-dimethylaminopropane (DODMA), 1,2-dilinoleyloxy-3-dimethylaminopropane (DLinDMA), 2-[2,2-bis[(9Z,12Z)-octadeca-9,12-dienyl]-1,3-dioxolan-4-yl]-N,N-dimethylethaneamine (DLin-KC2-DMA), (6Z,9Z,28Z,31Z)-heptatriaconta-6,9,28,31-tetraen-19-yl 4-(dimethylamino)butanoate (DLin-MC3-DMA or MC3), heptadecan-9-yl 8-((2-hydroxyethyl) (8-(nonyloxy)-8-oxooctyl)amino)octanoate (Lipid5), heptadecan-9-yl 8-((2-hydroxyethyl) (6-oxo-6-(undecyloxy)hexyl) amino)octanoate (Lipid8), a compound represented by the following formula (1):

[Chem. 2]

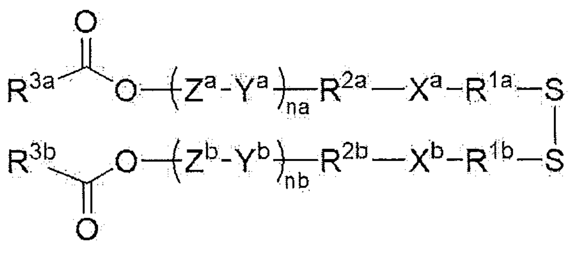
(1)

(in the formula (1),

$R^{1a}$ and $R^{1b}$ are each independently an alkylene group having 1 to 6 carbon atoms,
$X^a$ and $X^b$ are each independently an acyclic alkyl tertiary amino group having 1 to 6 carbon atoms and 1 tertiary amino group, or a cyclic alkylene tertiary amino group having 2 to 5 carbon atoms and 1 or 2 tertiary amino groups,
$R^{2a}$ and $R^{2b}$ are each independently an alkylene group having 1 to 8 carbon atoms or an oxydialkylene group having 2 to 8 carbon atoms,
$Y^a$ and $Y^b$ are each independently an ester bond, an amide bond, a carbamate bond, an ether bond, or a urea bond,
$Z^a$ and $Z^b$ are each independently a divalent group derived from an aromatic compound having 3 to 16 carbon atoms and at least one aromatic ring, and optionally having a hetero atom,
na and nb are each independently 0 or 1,
$R^{3a}$ and $R^{3b}$ are each independently a residue derived from a reaction product of a liposoluble vitamin having a hydroxyl group, and succinic anhydride or glutaric anhydride, a residue derived from a reaction product of a sterol derivative having a hydroxyl group, and succinic anhydride or glutaric anhydride, an aliphatic hydrocarbon group having 1 to 40 carbon atoms, an alkyl group having a cyclopropane ring and having 3 to 40 carbon atoms, or a group represented by the formula (3c):

$$R^{3c}\text{-O-CO-}(CH_2)_a\text{-}^* \qquad (3c)$$

(in the formula (3c),
* is a bonding position,
$R^{3c}$ is an aliphatic hydrocarbon group having 2 to 20 carbon atoms, and
a is an integer of 2 to 10))

(hereinafter sometimes to be referred to as "ionic lipid (1)").

The ionic lipid used in the present invention is preferably ionic lipid (1). Only one type of ionic lipid (1) may be used, or two or more types thereof may be used in combination. Each group in ionic lipid (1) is explained in order in the following.

$R^{1a}$ and $R^{1b}$ are each independently an alkylene group having 1 to 6 carbon atoms, and may be linear or branched, preferably linear. The carbon number of the alkylene group is preferably 1 to 4, more preferably 1 or 2. Specific examples of the alkylene group having 1 to 6 carbon atoms include methylene group, ethylene group, trimethylene group, isopropylene group, tetramethylene group, isobutylene group, pentamethylene group, neopentylene group and the like. Preferably, $R^{1a}$ and $R^{1b}$ are each independently a methylene group, an ethylene group, a trimethylene group, an isopropylene group or a tetramethylene group, most preferably an ethylene group.

$R^{1a}$ may be the same as or different from $R^{1b}$, and $R^{1a}$ is preferably the same group as $R^{1b}$.

$X^a$ and $X^b$ are each independently an acyclic alkyl tertiary amino group having 1 to 6 carbon atoms and 1 tertiary amino group, or a cyclic alkylene tertiary amino group having 2 to 5 carbon atoms and 1 or 2 tertiary amino groups, preferably each independently a cyclic alkylene tertiary amino group having 2 to 5 carbon atoms and 1 or 2 tertiary amino groups.

The alkyl group having 1 to 6 carbon atoms in the acyclic alkyl tertiary amino group having 1 to 6 carbon atoms and 1 tertiary amino group may be linear, branched or cyclic. The alkyl group preferably has a carbon number of 1 to 3. Specific examples of the alkyl group having 1 to 6 carbon atoms include methyl group, ethyl group, propyl group, isopropyl group, n-butyl group, sec-butyl group, isobutyl group, tert-butyl group, pentyl group, isopentyl group, neopentyl group, tert-pentyl group, 1,2-dimethylpropyl group, 2-methylbutyl group, 2-methylpentyl group, 3-methylpentyl group, 2,2-dimethylbutyl group, 2,3-dimethylbutyl group, cyclohexyl group and the like, preferably methyl group, ethyl group, propyl group or isopropyl group, most preferably methyl group.

A preferable specific structure of the acyclic alkyl tertiary amino group having 1 to 6 carbon atoms and 1 tertiary amino group is represented by $X^1$.

[Chem. 3]

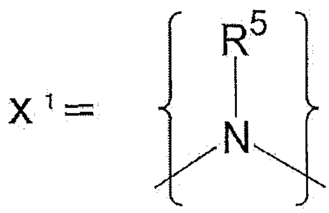

$R^5$ in $X^1$ is an alkyl group having 1 to 6 carbon atoms, which may be linear, branched or cyclic. The alkyl group preferably has a carbon number of 1 to 3. Specific examples of the alkyl group having 1 to 6 carbon atoms include methyl

group, an ethyl group, a propyl group, an isopropyl group, n-butyl group, a sec-butyl group, an isobutyl group, a tert-butyl group, a pentyl group, isopentyl group, neopentyl group, tert-pentyl group, 1,2-dimethylpropyl group, 2-methylbutyl group, 2-methylpentyl group, 3-methylpentyl group, 2,2-dimethylbutyl group, 2,3-dimethylbutyl group, cyclohexyl group and the like. $R^5$ is preferably a methyl group, an ethyl group, a propyl group or an isopropyl group, most preferably a methyl group.

The carbon number of the cyclic alkylene tertiary amino group having 2 to 5 carbon atoms and 1 or 2 tertiary amino groups is preferably 4 or 5. The cyclic alkylene tertiary amino group having 2 to 5 carbon atoms and 1 or 2 tertiary amino groups is specifically an aziridinediyl group, an azetidinediyl group, a pyrrolidinediyl group, a piperidinediyl group, an imidazolidinediyl group, or a piperazinediyl group, preferably a pyrrolidinediyl group, a piperidinediyl group, or a piperazinediyl group, most preferably a piperidinediyl group.

A preferable specific structure of the cyclic alkylene tertiary amino group having 2 to 5 carbon atoms and 1 tertiary amino group is represented by $X^2$.

[Chem. 4]

$X^2 =$ 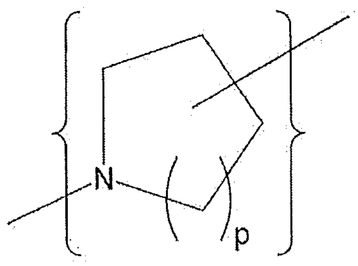

The p of $X^2$ is 1 or 2. When p is 1, $X^2$ is a pyrrolidinediyl group, and when p is 2, $X^2$ is a piperazinediyl group. Preferably, p is 2.

A preferable specific structure of the cyclic alkylene tertiary amino group having 2 to 5 carbon atoms and two tertiary amino groups is represented by $X^3$.

[Chem. 5]

$X^3 =$ 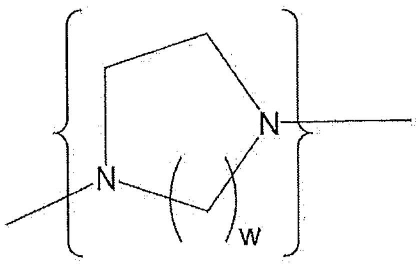

The w of $X^3$ is 1 or 2. When w is 1, $X^3$ is an imidazolidinediyl group, and when w is 2, $X^3$ is a piperadinediyl group.

$X^a$ may be the same as or different from $X^b$, and $X^a$ is preferably the same group as $X^b$.

$R^{2a}$ and $R^{2b}$ are each independently an alkylene group having 1 to 8 carbon atoms or an oxydialkylene group having 2 to 8 carbon atoms, preferably each independently an alkylene group having 1 to 8 carbon atoms.

The alkylene group having 1 to 8 carbon atoms may be linear or branched, preferably linear. The number of carbons contained in the alkylene group is preferably not more than 6, most preferably not more than 4. Specific examples of the alkylene group having 1 to 8 carbon atoms include methylene group, ethylene group, trimethylene group, isopropylene group, tetramethylene group, isobutylene group, pentamethylene group, hexamethylene group, heptamethylene group, octamethylene group, and the like, preferably methylene group, ethylene group, trimethylene group, and tetramethylene group, most preferably ethylene group.

In the present specification, the "oxydialkylene group having 2 to 8 carbon atoms" means an alkylene group (alkylene-O-alkylene, in other words, an "alkyleneoxyalkylene group") via an ether bond, in which the total number of carbon atoms in the two alkylene groups present is 8 or less. The two alkylene present may be the same or different, preferably the same. Specific examples of the oxydialkylene group having 2 to 8 carbon atoms include oxydimethylene group, oxydiethylene group, oxydi(trimethylene) group (i.e., trimethyleneoxytrimethylene group), oxydi(tetramethylene) group (i.e., tetramethyleneoxytetramethylene group) and the like. It is preferably an oxydimethylene group, an oxydiethylene group, an oxydi(trimethylene) group, most preferably an oxydiethylene group.

$R^{2a}$ may be the same as or different from $R^{2b}$, and $R^{2a}$ is preferably the same group as $R^{2b}$.

$Y^a$ and $Y^b$ are each independently an ester bond, an amide bond, a carbamate bond, an ether bond or a urea

bond, preferably each independently an ester bond, an amide bond or a carbamate bond, more preferably each independently an ester bond or an amide bond, most preferably each an ester bond. The direction of the bond of $Y^a$ and $Y^b$ is not limited. When $Y^a$ and $Y^b$ are ester bonds, the structure of $-Z^a-CO-O-R^{2a}-$ or $-Z^b-CO-O-R^{2b}-$ is preferably shown.

$Y^a$ may be the same as or different from $Y^b$, and $Y^a$ is preferably the same group as $Y^b$.

$Z^a$ and $Z^b$ are each independently a divalent group derived from an aromatic compound having 3 to 16 carbon atoms and at least one aromatic ring, and optionally having a hetero atom. The number of carbons contained in the aromatic compound is preferably 6 to 12, most preferably 6 or 7. The aromatic ring contained in the aromatic compound is preferably one.

As the kind of the aromatic ring contained in the aromatic compound having 3 to 16 carbon atoms, benzene ring, naphthalene ring, and anthracene ring can be mentioned for aromatic hydrocarbon ring, and imidazole ring, a pyrazole ring, oxazole ring, an isoxazole ring, thiazole ring, isothiazole ring, triazine ring, a pyrrole ring, furan ring, thiophene ring, a pyrimidine ring, a pyridazine ring, a pyrazine ring, a pyridine ring, purine ring, pteridine ring, benzimidazole ring, indole ring, benzofuran ring, quinazoline ring, phthalazine ring, quinoline ring, isoquinoline ring, coumarin ring, chromone ring, benzodiazepine ring, phenoxazine ring, phenothiazine ring, acridine ring and the like can be mentioned for aromatic heterocycle. It is preferably benzene ring, naphthalene ring, or anthracene ring, most preferably benzene ring.

The aromatic ring may have a substituent. Examples of the substituent include acyl group having 2 to 4 carbon atoms, alkoxycarbonyl group having 2 to 4 carbon atoms, alkylcarbamoyl group having 2 to 4 carbon atoms, acyloxy group having 2 to 4 carbon atoms, acylamino group having 2 to 4 carbon atoms, alkoxycarbonylamino group having 2 to 4 carbon atoms, fluorine atom, chlorine atom, bromine atom, iodine atom, alkylsulfanyl group having 1 to 4 carbon atoms, alkylsulfonyl group having 1 to 4 carbon atoms, arylsulfonyl group having 6 to 10 carbon atoms, nitro group, trifluoromethyl group, a cyano group, alkyl group having 1 to 4 carbon atoms, ureido group, alkoxy group having 1 to 4 carbon atoms, aryl group having 6 to 10 carbon atoms, aryloxy group having 6 to 10 carbon atoms, and the like. Preferable examples include acetyl group, methoxycarbonyl group, methylcarbamoyl group, acetoxy group, acetamido group, methoxycarbonylamino group, fluorine atom, chlorine atom, bromine atom, iodine atom, methylsulfanyl group, phenylsulfonyl group, nitro group, trifluoromethyl group, cyano group, methyl group, ethyl group, propyl group, isopropyl group, tert-butyl group, ureido group, methoxy group, ethoxy group, propoxy group, isopropoxy group, tert-butoxy group, phenyl group, phenoxy group and the like.

A preferable specific structure of $Z^a$ and $Z^b$ is $Z^1$.

[Chem. 6]

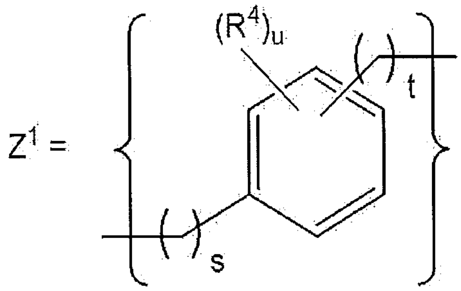

wherein s is an integer of 0 to 3, t is an integer of 0 to 3, u is an integer of 0 to 4, and $R^4$ in the number of u are each independently a substituent. In the present specification, "s is 0" mean that $(CH_2)_s$ in $Z^1$ is absent, "t is 0" means that $(CH_2)_t$ in $Z^1$ is absent, and "u is 0" means that $(R^4)_u$ in $Z^1$ is absent.

The s for $Z^1$ is preferably an integer of 0 or 1, more preferably 0.

The t for $Z^1$ is preferably an integer of 0 to 2, more preferably 1.

The u for $Z^1$ is preferably an integer of 0 to 2, more preferably an integer of 0 or 1.

The $R^4$ for $Z^1$ is a substituent of an aromatic ring (benzene ring) contained in the aromatic compound having 3 to 16 carbon atoms which does not inhibit the reaction in the synthesis process of the ionic lipid. Examples of the substituent include acyl group having 2 to 4 carbon atoms, alkoxycarbonyl group having 2 to 4 carbon atoms, alkylcarbamoyl group having 2 to 4 carbon atoms, acyloxy group having 2 to 4 carbon atoms, acylamino group having 2 to 4 carbon atoms, alkoxycarbonylamino group having 2 to 4 carbon atoms, fluorine atom, chlorine atom, bromine atom, iodine atom, alkylsulfanyl group having 1 to 4 carbon atoms, alkylsulfonyl group having 1 to 4 carbon atoms, arylsulfonyl group having 6 to 10 carbon atoms, nitro group, trifluoromethyl group, a cyano group, alkyl group having 1 to 4 carbon atoms, ureido group, alkoxy group having 1 to 4 carbon atoms, aryl group having 6 to 10 carbon atoms, aryloxy group having 6 to 10 carbon atoms, and the like. Preferable examples include acetyl group, methoxycarbonyl group, methylcarbamoyl group, acetoxy group, acetamido group, methoxycarbonylamino group, fluorine atom, chlorine atom, bromine atom, iodine atom,

methylsulfanyl group, phenylsulfonyl group, nitro group, trifluoromethyl group, cyano group, methyl group, ethyl group, propyl group, isopropyl group, tert-butyl group, ureido group, methoxy group, ethoxy group, propoxy group, isopropoxy group, tert-butoxy group, phenyl group, phenoxy group and the like. When $R^4$ is present in plurality, each $R^4$ may be the same or different.

$Z^a$ may be the same as or different from $Z^b$, and $Z^a$ is preferably the same group as $Z^b$.

na and nb are each independently 0 or 1. In the present specification, "na is 0" means that $(Z^a\text{-}Y^a)_{na}$ in the formula (1) is absent, and "nb is 0" means that $(Z^b\text{-}Y^b)_{nb}$ in the formula (1) is absent.

na may be the same as or different from nb, and na is preferably the same as nb.

$R^{3a}$ and $R^{3b}$ are each independently a residue derived from a reaction product of a liposoluble vitamin having a hydroxyl group, and succinic anhydride or glutaric anhydride, a residue derived from a reaction product of a sterol derivative having a hydroxyl group, and succinic anhydride or glutaric anhydride, an aliphatic hydrocarbon group having 1 to 40 carbon atoms, an alkyl group having a cyclopropane ring and having 3 to 40 carbon atoms, or a group represented by the formula (3c):

$$R^{3c}\text{-O-CO-}(CH_2)_a\text{-}* \qquad (3c)$$

(in the formula (3c),

* is a bonding position,
$R^{3c}$ is an aliphatic hydrocarbon group having 2 to 20 carbon atoms, and
a is an integer of 2 to 10).

$R^{3a}$ and $R^{3b}$ are preferably each independently a residue derived from a reaction product of a liposoluble vitamin having a hydroxyl group, and succinic anhydride or glutaric anhydride, or an aliphatic hydrocarbon group having 12 to 22 carbon atoms, or a group represented by the above-mentioned formula (3c), more preferably a residue derived from a reaction product of a liposoluble vitamin having a hydroxyl group, and succinic anhydride or glutaric anhydride, or an aliphatic hydrocarbon group having 12 to 22 carbon atoms, most preferably an aliphatic hydrocarbon group having 12 to 22 carbon atoms.

The residue derived from a reaction product of a liposoluble vitamin having a hydroxyl group, and succinic anhydride or glutaric anhydride is a group having a structure in which the hydroxyl group of a liposoluble vitamin having a hydroxyl group is replaced by $*\text{-O-CO-}CH_2\text{-}CH_2\text{-}$ or $*\text{-O-CO-}CH_2\text{-}CH_2\text{-}CH_2\text{-}$ (in the aforementioned formulas, * is the bonding position with the liposoluble vitamin). The residue derived from a reaction product of a sterol derivative having a hydroxyl group, and succinic anhydride or glutaric anhydride is a group having a structure in which the hydroxyl group of a sterol derivative having a hydroxyl group is replaced by $*\text{-O-CO-}CH_2\text{-}CH_2\text{-}$ or $*\text{-O-CO-}CH_2\text{-}CH_2\text{-}CH_2\text{-}$ (in the aforementioned formulas, * is the bonding position with the sterol derivative).

The liposoluble vitamin having a hydroxyl group is, for example, retinol, ergosterol, 7-dehydrocholesterol, calciferol, cholecalciferol, dihydroergocalciferol, dihydrotachysterol, tocopherol, tocotrienol and the like. Preferred example of the liposoluble vitamin having a hydroxyl group is tocopherol.

Examples of the sterol derivative having a hydroxyl group include cholesterol, cholestanol, stigmasterol, β-sitosterol, lanosterol, and ergosterol and the like, preferably cholesterol or cholestanol.

The aliphatic hydrocarbon group having 1 to 40 carbon atoms may be linear or branched. The aliphatic hydrocarbon group may be saturated or unsaturated. In the case of an unsaturated aliphatic hydrocarbon group, the aliphatic hydrocarbon group generally contains 1 to 6, preferably 1 to 3, more preferably 1 or 2 unsaturated bonds. While the unsaturated bond includes a carbon-carbon double bond and a carbon-carbon triple bond, it is preferably a carbon-carbon double bond. The aliphatic hydrocarbon group has a carbon number of preferably 12 to 22, more preferably 13 to 19, most preferably 13 to 17. While the aliphatic hydrocarbon group includes an alkyl group, an alkenyl group, an alkynyl group and the like, it is preferably an alkyl group or an alkenyl group. Specific examples of the aliphatic hydrocarbon group having 1 to 40 carbon atoms include methyl group, ethyl group, propyl group, isopropyl group, n-butyl group, sec-butyl group, isobutyl group, tert-butyl group, pentyl group, isopentyl group, neopentyl group, tert-pentyl group, hexyl group, heptyl group, octyl group, nonyl group, decyl group, undecyl group, dodecyl group, tridecyl group, tetradecyl group, pentadecyl group, hexadecyl group, heptadecyl group, octadecyl group, nonadecyl group, icosyl group, henicosyl group, docosyl group, tricosyl group, tetracosyl group, pentacosyl group, hexacosyl group, heptacosyl group, octacosyl group, nonacosyl group, triacontyl group, tetracontyl group, dodecenyl group, tridecenyl group, tetradecenyl group, pentadecenyl group, hexadecenyl group, heptadecenyl group, octadecenyl group, nonadecenyl group, icosenyl group, henicosenyl group, docosenyl group, dodecadienyl group, tridecadienyl group, tetradecadienyl group, pentadecadienyl group, hexadecadienyl group, heptadecadienyl group, octadecadienyl group, nonadecadienyl group, icosadienyl group, henicosadienyl group, docosadienyl group, octadecatrienyl group, icosatrienyl group, icosatetraenyl group, icosapentaenyl group, docosahexaenyl group, isostearyl group, 1-hexylheptyl group, 1-hexylnonyl group, 1-octylnonyl group, 1-octylundecyl group, 1-decylundecyl group and the like. The aliphatic hydrocarbon group having 1 to 40 carbon atoms is preferably tridecyl group, pentadecyl group, heptadecyl group, nonadecyl group, heptadecenyl group, heptadecadienyl group, or 1-hexylnonyl group, particularly preferably tridecyl group, heptadecyl group, heptadecenyl group, or heptadecadienyl group.

In one embodiment of the present invention, the aliphatic hydrocarbon group having 1 to 40 carbon atoms (preferably 12 to 22 carbon atoms) represented by $R^{3a}$ or $R^{3b}$ is derived from fatty acid. In this case, the carbonyl carbon derived from fatty acid is contained in -CO-O- in the formula (1). A specific example of the aliphatic hydrocarbon group is a heptadecadienyl group when linoleic acid is used as the fatty acid, and heptadecenyl group when oleic acid is used as the fatty acid.

The alkyl group having a cyclopropane ring and having 3 to 40 carbon atoms for $R^{3a}$ or $R^{3b}$ means an alkyl group having at least one cyclopropane ring in the alkyl chain and having 3 to 40 carbon atoms. The number of carbon atoms in the alkyl group is 3 to 40, and does not include the number of carbon atoms in the cyclopropane ring. The number of cyclopropane rings in the alkyl group is preferably one. The alkyl group having a cyclopropane ring and having 3 to 40 carbon atoms for $R^{3a}$ or $R^{3b}$ is preferably a group represented by the formula (4):

[Chem. 7]

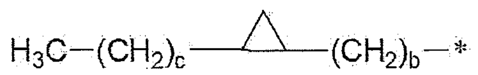 (4)

(in the formula (4),

- * is a bonding position, and
- b and c are each independently an integer and the total of b and c is 2 to 39). Preferably, b is an integer of 1 to 20 and c is an integer of 1 to 19. b is more preferably an integer of 2 to 18, further preferably an integer of 3 to 17, particularly preferably an integer of 4 to 12. c is more preferably an integer of 3 to 15, further preferably an integer of 3 to 11, particularly preferably an integer of 3 to 9. Examples of the group represented by the formula (4) include 7-(2-octylcyclopropyl)heptyl and the like.

In the formula (3c), the aliphatic hydrocarbon group having 2 to 20 carbon atoms for $R^{3c}$ may be linear or branched. The aliphatic hydrocarbon group may be saturated or unsaturated. In the case of an unsaturated aliphatic hydrocarbon group, the aliphatic hydrocarbon group generally contains 1 to 6, preferably 1 to 3, more preferably 1 or 2 unsaturated bonds. While the unsaturated bond includes a carbon-carbon double bond and a carbon-carbon triple bond, it is preferably a carbon-carbon double bond. The aliphatic hydrocarbon group has a carbon number of preferably 8 to 20, more preferably 9 to 19, further preferably 13 to 19, most preferably 13 to 17. While the aliphatic hydrocarbon group includes an alkyl group, an alkenyl group, an alkynyl group and the like, it is preferably an alkyl group or an alkenyl group, more preferably alkyl group. Specific examples of the aliphatic hydrocarbon group having 2 to 20 carbon atoms include methyl group, ethyl group, propyl group, isopropyl group, n-butyl group, sec-butyl group, isobutyl group, tert-butyl group, pentyl group, isopentyl group, neopentyl group, tert-pentyl group, hexyl group, heptyl group, octyl group, nonyl group, decyl group, undecyl group, dodecyl group, tridecyl group, tetradecyl group, pentadecyl group, hexadecyl group, heptadecyl group, octadecyl group, nonadecyl group, icosyl group, dodecenyl group, tridecenyl group, tetradecenyl group, pentadecenyl group, hexadecenyl group, heptadecenyl group, octadecyl group, nonadecenyl group, icosenyl group, henicosenyl group, docosenyl group, dodecadienyl group, tridecadienyl group, tetradecadienyl group, pentadecadienyl group, hexadecadienyl group, heptadecadienyl group, octadecadienyl group, nonadecadienyl group, icosadienyl group, icosatrienyl group, icosatetraenyl group, icosapentaenyl group, isostearyl group, 1-hexylheptyl group, 1-ethylnonyl group, 1-butylnonyl group, 1-hexylnonyl group, 1-octylnonyl group, 1-octylundecyl group, 3-octylundecyl group, and the like. The aliphatic hydrocarbon group having 2 to 20 carbon atoms is preferably tridecyl group, pentadecyl group, heptadecyl group, nonadecyl group, heptadecenyl group, heptadecadienyl group, or 1-hexylnonyl group, particularly preferably tridecyl group, heptadecyl group, heptadecenyl group, or heptadecadienyl group.

In the formula (3c), a is preferably an integer of 3 to 9, more preferably an integer of 3 to 7, further preferably an integer of 5 to 7, and most preferably 5 or 7.

$R^{3a}$ may be the same as or different from $R^{3b}$, and $R^{3a}$ is preferably the same group as $R^{3b}$.

In one embodiment of the present invention, $R^{1a}$ is the same as $R^{1b}$, $X^a$ is the same as $X^b$, $R^{2a}$ is the same as $R^{2b}$, $Y^a$ is the same as $Y^b$, $Z^a$ is the same as $Z^b$, and $R^{3a}$ is the same as $R^{3b}$.

Preferable examples of the ionic lipid (1) include the following ionic lipids.

[Ionic lipid (1-1a)]

Ionic lipid (1) wherein

$R^{1a}$ and $R^{1b}$ are each independently an alkylene group having 1 to 6 carbon atoms (e.g., methylene group, ethylene group);
$X^a$ and $X^b$ are each independently an acyclic alkyl tertiary amino group having 1 to 6 carbon atoms and 1 tertiary amino group (e.g., $-N(CH_3)-$), or a cyclic alkylene tertiary amino group having 2 to 5 carbon atoms and 1 to 2 tertiary amino groups (e.g., piperidinediyl group);
$R^{2a}$ and $R^{2b}$ are each independently an alkylene group having 1 to 8 carbon atoms (e.g., methylene group, ethylene group, trimethylene group);
$Y^a$ and $Y^b$ are each independently an ester bond or an amide bond;
$Z^a$ and $Z^b$ are each independently a divalent group derived from an aromatic compound having 3 to 16 carbon atoms and at least one aromatic ring, and optionally having a hetero atom (e.g., $-C_6H_4-CH_2-$, $-CH_2-C_6H_4-CH_2-$);
na and nb are each independently 0 or 1;
$R^{3a}$ and $R^{3b}$ are each independently a residue derived from a reaction product of a liposoluble vitamin having a hydroxyl group (e.g., tocopherol), and succinic anhydride or glutaric anhydride, an aliphatic hydrocarbon group having 12 to 22 carbon atoms (e.g., heptadecenyl group, heptadecadienyl group, 1-hexylnonyl group), or a group represented by the formula (3c):

$$R^{3c}\text{-O-CO-}(CH_2)_a\text{-*} \qquad (3c)$$

(in the formula (3c),
* is a bonding position,
$R^{3c}$ is an aliphatic hydrocarbon group having 2 to 20 carbon atoms, and
a is an integer of 2 to 10).

[Ionic lipid (1-1b)]

Ionic lipid (1) wherein

$R^{1a}$ and $R^{1b}$ are each independently an alkylene group having 1 to 6 carbon atoms (e.g., methylene group, ethylene group);
$X^a$ and $X^b$ are each independently an acyclic alkyl tertiary amino group having 1 to 6 carbon atoms and 1 tertiary amino group (e.g., $-N(CH_3)-$), or a cyclic alkylene tertiary amino group having 2 to 5 carbon atoms and 1 or 2 tertiary amino groups (e.g., piperidinediyl group);
$R^{2a}$ and $R^{2b}$ are each independently an alkylene group having 1 to 8 carbon atoms (e.g., methylene group, ethylene group, trimethylene group);
$Y^a$ and $Y^b$ are each independently an ester bond or an amide bond;
$Z^a$ and $Z^b$ are each independently a divalent group derived from an aromatic compound having 3 to 16 carbon atoms and at least one aromatic ring, and optionally having a hetero atom (e.g., $-C_6H_4-CH_2-$, $-CH_2-C_6H_4-CH_2-$);
na and nb are each independently 0 or 1;
$R^{3a}$ and $R^{3b}$ are each independently a residue derived from a reaction product of a liposoluble vitamin having a hydroxyl group (e.g., tocopherol), and succinic anhydride or glutaric anhydride, or an aliphatic hydrocarbon group having 12 to 22 carbon atoms (e.g., heptadecenyl group, heptadecadienyl group, 1-hexylnonyl group).

[Ionic lipid (1-2a)]

Ionic lipid (1) wherein

$R^{1a}$ and $R^{1b}$ are each independently an alkylene group having 1 to 4 carbon atoms (e.g., methylene group, ethylene group);
$X^a$ and $X^b$ are each independently an acyclic alkyl tertiary amino group having 1 to 3 carbon atoms and 1 tertiary amino group (e.g., $-N(CH_3)-$), or a cyclic alkylene tertiary amino group having 2 to 5 carbon atoms and 1 tertiary amino group (e.g., piperidinediyl group);
$R^{2a}$ and $R^{2b}$ are each independently an alkylene group having 6 or less carbon atoms (e.g., methylene group, ethylene group, trimethylene group);
$Y^a$ and $Y^b$ are each independently an ester bond or an amide bond;

$Z^a$ and $Z^b$ are each independently a divalent group derived from an aromatic compound having 6 to 12 carbon atoms and one aromatic ring, and optionally having a hetero atom (e.g., $-C_6H_4-CH_2-$, $-CH_2-C_6H_4-CH_2-$) ;
na and nb are each independently 0 or 1;
$R^{3a}$ and $R^{3b}$ are each independently a residue derived from a reaction product of a liposoluble vitamin having a hydroxyl group (e.g., tocopherol), and succinic anhydride, or an aliphatic hydrocarbon group having 13 to 19 carbon atoms (e.g., heptadecenyl group, heptadecadienyl group, 1-hexylnonyl group), or a group represented by the formula (3c):

$$R^{3c}\text{-O-CO-}(CH_2)_a\text{-*} \qquad (3c)$$

(in the formula (3c),
* is a bonding position,
$R^{3c}$ is an aliphatic hydrocarbon group having 8 to 20 carbon atoms, and
a is an integer of 3 to 9.)

[Ionic lipid (1-2b)]

Ionic lipid (1) wherein

$R^{1a}$ and $R^{1b}$ are each independently an alkylene group having 1 to 4 carbon atoms (e.g., methylene group, ethylene group);
$X^a$ and $X^b$ are each independently an acyclic alkyl tertiary amino group having 1 to 3 carbon atoms and 1 tertiary amino group (e.g., $-N(CH_3)-$), or a cyclic alkylene tertiary amino group having 2 to 5 carbon atoms and 1 tertiary amino groups (e.g., piperidinediyl group);
$R^{2a}$ and $R^{2b}$ are each independently an alkylene group having 6 or less carbon atoms (e.g., methylene group, ethylene group, trimethylene group);
$Y^a$ and $Y^b$ are each independently an ester bond or an amide bond;
$Z^a$ and $Z^b$ are each independently a divalent group derived from an aromatic compound having 6 to 12 carbon atoms and one aromatic ring, and optionally having a hetero atom (e.g., $-C_6H_4-CH_2-$, $-CH_2-C_6H_4-CH_2-$) ;
na and nb are each independently 0 or 1; and
$R^{3a}$ and $R^{3b}$ are each independently a residue derived from a reaction product of a liposoluble vitamin having a hydroxyl group (e.g., tocopherol), and succinic anhydride, or an aliphatic hydrocarbon group having 13 to 19 carbon atoms (e.g., heptadecenyl group, heptadecadienyl group, 1-hexylnonyl group).

[Ionic lipid (1-3a)]

Ionic lipid (1) wherein

$R^{1a}$ and $R^{1b}$ are each independently an alkylene group having 1 or 2 carbon atoms (i.e., methylene group or ethylene group);
$X^a$ and $X^b$ are each independently $X^1$:

[Chem. 8]

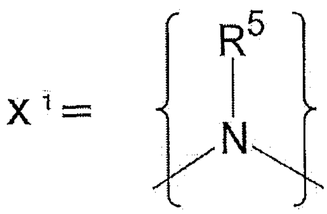

wherein $R^5$ is an alkyl group having 1 to 3 carbon atoms (e.g., a methyl group)), or $X^2$:

[Chem. 9]

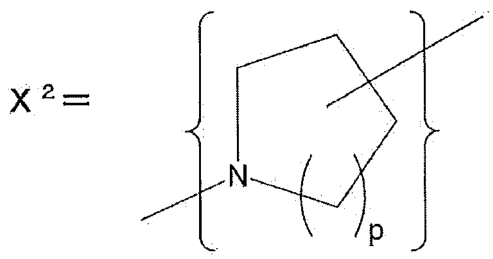

wherein p is 1 or 2);

$R^{2a}$ and $R^{2b}$ are each independently an alkylene group having 4 or less carbon atoms (e.g., methylene group, ethylene group, trimethylene group);
$Y^a$ and $Y^b$ are each independently an ester bond or an amide bond;
$Z^a$ and $Z^b$ are each independently $Z^1$:

[Chem. 10]

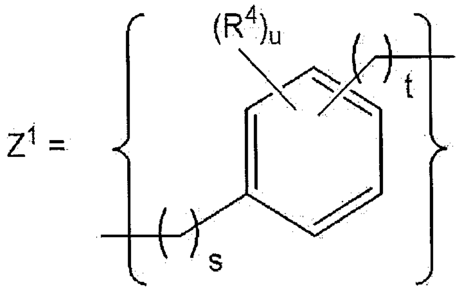

wherein s is an integer of 0 or 1, t is an integer of 0 to 2, u is an integer of 0 to 2 (preferably 0), $R^4$ in the number of u are each independently a substituent);
na and nb are each independently 0 or 1;
$R^{3a}$ and $R^{3b}$ are each independently a residue derived from a reaction product of a liposoluble vitamin having a hydroxyl group (e.g., tocopherol), and succinic anhydride, or an aliphatic hydrocarbon group having 13 to 17 carbon atoms (e.g., heptadecenyl group, heptadecadienyl group, 1-hexylnonyl group), or a group represented by the formula (3c):

$$R^{3c}\text{-O-CO-}(CH_2)_a\text{-*} \qquad (3c)$$

(in the formula (3c),
* is a bonding position,
$R^{3c}$ is an aliphatic hydrocarbon group having 13 to 19 carbon atoms (preferably aliphatic hydrocarbon group having 3 to 17 carbon atoms), and
a is an integer of 5 to 7 (preferably 5 or 7)).

[Ionic lipid (1-3b)]

Ionic lipid (1) wherein

$R^{1a}$ and $R^{1b}$ are each independently an alkylene group having 1 or 2 carbon atoms (i.e., methylene group or ethylene group);
$X^a$ and $X^b$ are each independently $X^1$:

[Chem. 11]

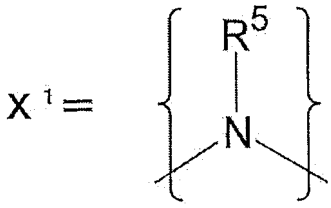

wherein $R^5$ is an alkyl group having 1 to 3 carbon atoms (e.g., a methyl group)), or $X^2$:

[Chem. 12]

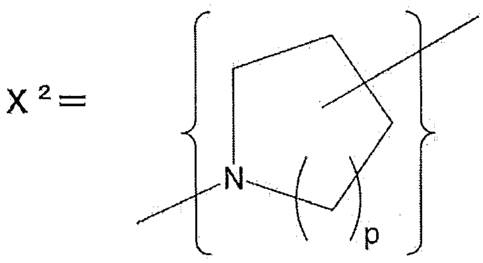

wherein p is 1 or 2);

$R^{2a}$ and $R^{2b}$ are each independently an alkylene group having 4 or less carbon atoms (e.g., methylene group, ethylene group, trimethylene group);
$Y^a$ and $Y^b$ are each independently an ester bond or an amide bond;
$Z^a$ and $Z^b$ are each independently $Z^1$:

[Chem. 13]

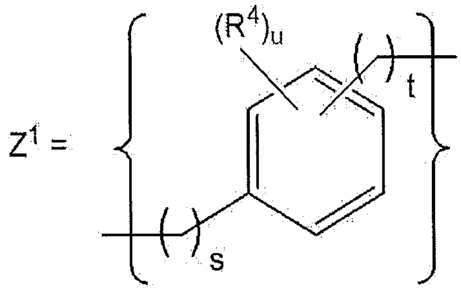

wherein s is an integer of 0 or 1, t is an integer of 0 to 2, u is an integer of 0 to 2 (preferably 0), $R^4$ in the number of u are each independently a substituent);
na and nb are each independently 0 or 1; and
$R^{3a}$ and $R^{3b}$ are each independently a residue derived from a reaction product of a liposoluble vitamin having a hydroxyl group (e.g., tocopherol), and succinic anhydride, or an aliphatic hydrocarbon group having 13 to 17 carbon atoms (e.g., heptadecenyl group, heptadecadienyl group, 1-hexylnonyl group).

Specific examples of ionic lipid (1) include SS-OP, SS-EC, O-Ph-P3C1, O-Ph-P4C1, O-Bn-P4C2, E-Ph-P4C2, L-Ph-P4C2, HD-Ph-P4C2, O-Ph-amide-P4C2, O-Ph-C3M, B-2, B-2-5, TS-P4C2, L-P4C2, and O-P4C2, described in the following Table 1-1 to Table 1-3.

[Table 1-1]

| name of ionic lipid | structure |
|---|---|
| SS-OP | 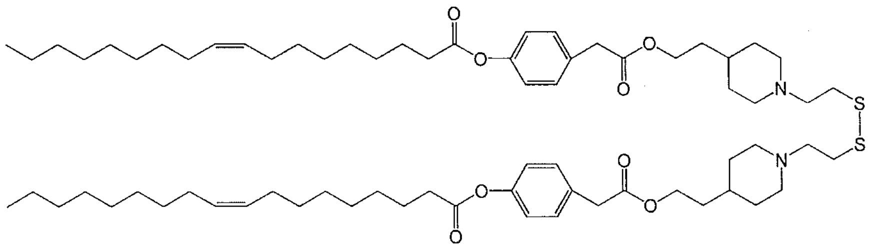 |
| SS-EC | 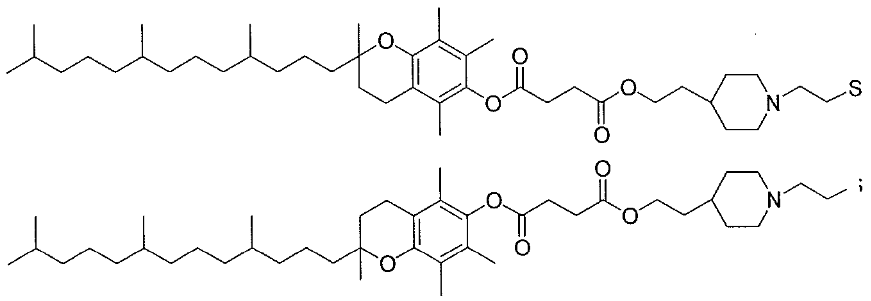 |
| O-Ph-P3C1 | 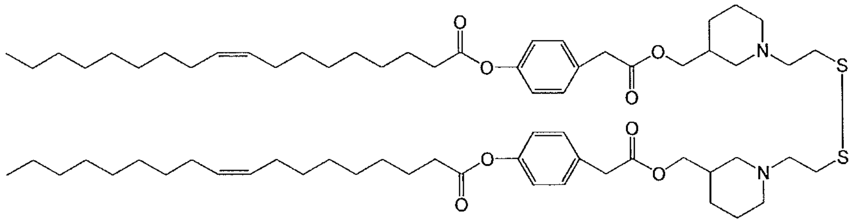 |
| O-Ph-P4C1 | 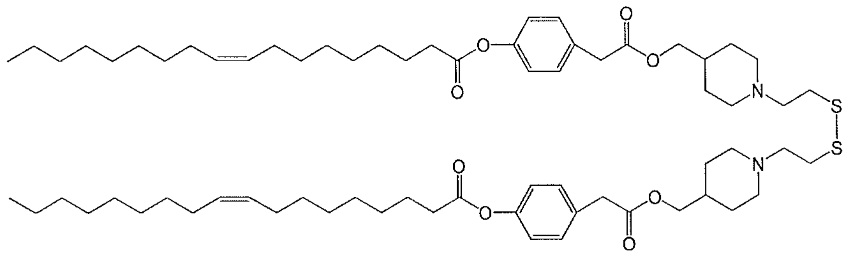 |
| O-Bn-P4C2 | 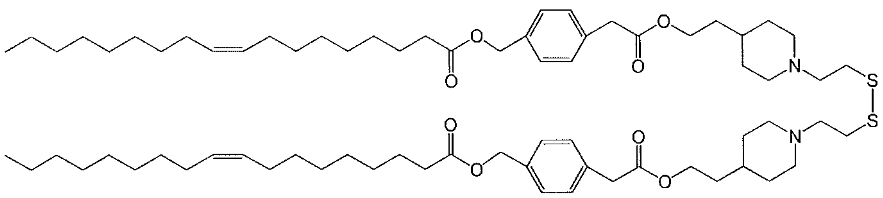 |

[Table 1-2]

| name of ionic lipid | structure |
|---|---|
| E-Ph-P4C2 (or SS-EP) | 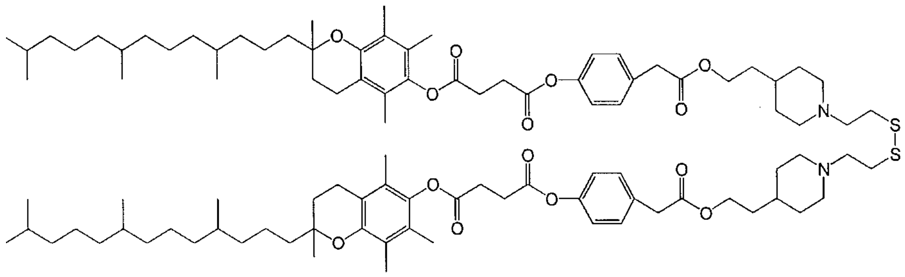 |
| L-Ph-P4C2 | 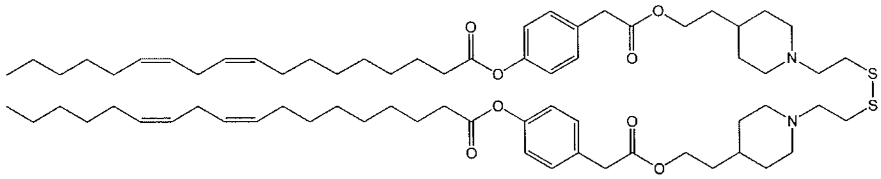 |
| HD-Ph-P4C2 | 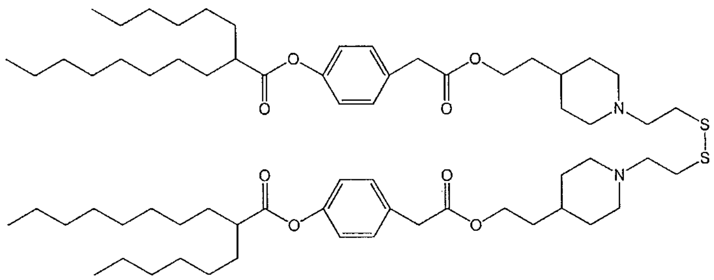 |
| O-Ph-amide-P4C2 | 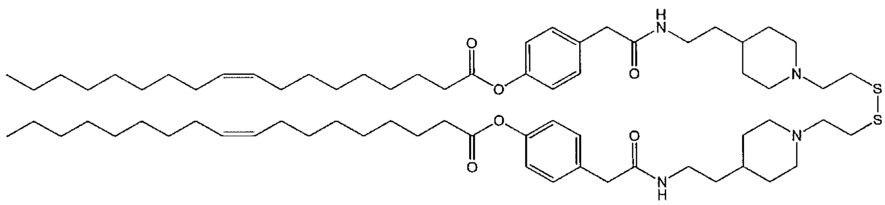 |
| O-Ph-C3M | 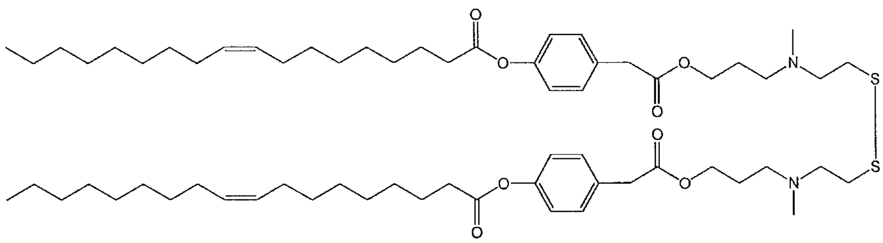 |

[Table 1-3]

| name of ionic lipid | structure |
|---|---|
| B-2 | 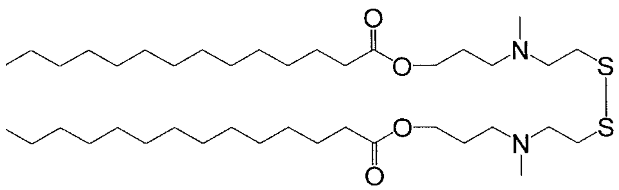 |

(continued)

| name of ionic lipid | structure |
|---|---|
| B-2-5 | 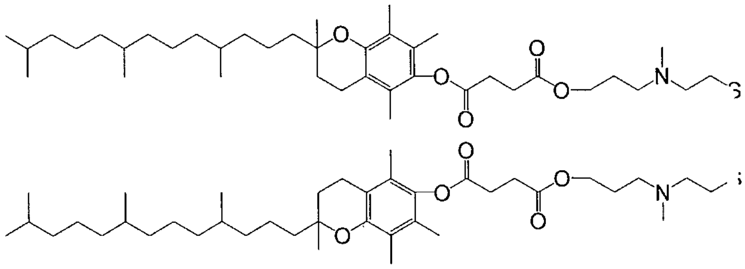 |
| TS-P4C2 | 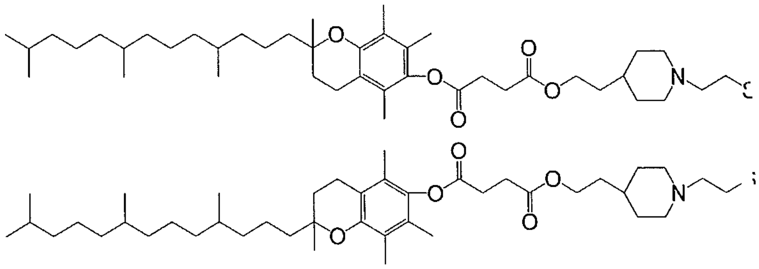 |
| L-P4C2 | 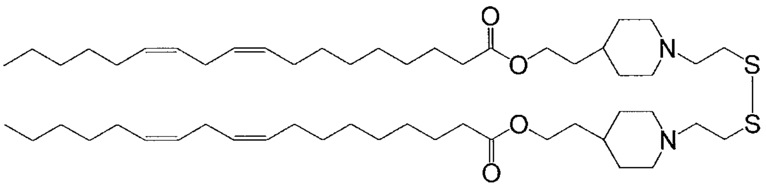 |
| O-P4C2 (or SS-OC) | 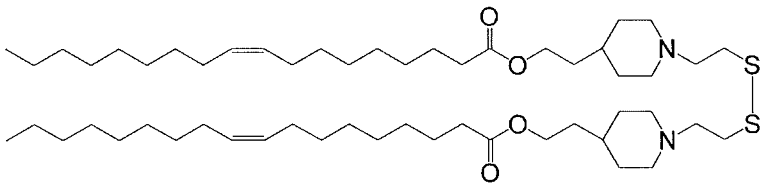 |
| compound (5) | 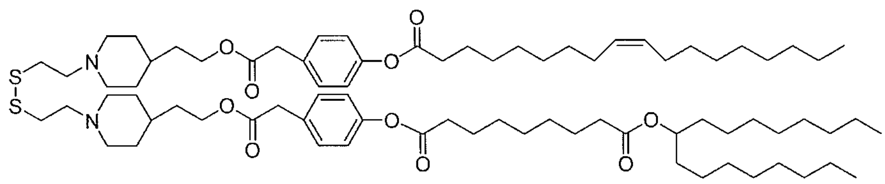 |

Specific examples of ionic lipid (1) are Lipid 1 to Lipid 20 described in WO2021/195529A2.

Ionic lipid (1) is preferably at least one selected from the group consisting of SS-OP, SS-EC, O-Ph-P3C1, O-Ph-P4C1, O-Bn-P4C2, E-Ph-P4C2, L-Ph-P4C2, HD-Ph-P4C2, O-Ph-amide-P4C2, O-Ph-C3M, B-2, B-2-5, TS-P4C2, L-P4C2, O-P4C2, and compound (5), more preferably at least one selected from the group consisting of SS-OP, SS-EC, and compound (5), further preferably SS-OP and/or SS-EC.

Ionic lipid (1) can be produced by a known method (e.g., methods described in WO2019/188867A1 (US2021/0023008A1), US9708628B2, WO2021/195529A2).

From the aspect of lipid solubility, the concentration of the ionic lipid in the alcohol solution is preferably 0.1 to 40 mM, more preferably 1 to 20 mM.

Sterol

The alcohol solution used in step (a) contains a sterol. Only one sterol may be used, or two or more sterols may be used in combination. Sterol is a component that regulates fluidity of the lipid membrane of lipid nanoparticles. Examples of the sterol include cholesterol, lanosterol, phytosterol, zymosterol, zymostenol, desmosterol, stigmastanol, dihydrolanosterol, and 7-dehydrocholesterol. Sterol is preferably at least one selected from the group consisting of cholesterol,

lanosterol, and phytosterol, and is further preferably cholesterol.

From the aspect of lipid solubility, the concentration of the sterol in the alcohol solution is preferably 0.1 to 40 mM, more preferably 0.5 to 20 mM.

From the aspect of the stability of the nucleic acid-encapsulating lipid nanoparticles and nucleic acid delivery efficiency, the molar ratio of the sterol to the ionic lipid (sterol/ionic lipid) used in step (a) is preferably 0.01 to 1.0, more preferably 0.1 to 0.9.

PEG lipid

The alcohol solution used in step (a) contains a PEG lipid. In the present specification, "PEG lipid" means a lipid having a polyethylene glycol (PEG) chain. Only one type of PEG lipid may be used or two or more types thereof may be used in combination. PEG lipids are used as stabilizers that coat the surface of lipid nanoparticles with hydrophilic PEG chains and suppress the aggregation of particles, or used to suppress the interaction between biological components and particles when administered to a living organism.

The PEG chain can have any molecular weight. In some embodiments, the PEG chain has a number average molecular weight of 200 to 10,000 and may be linear or branched. The number average molecular weight can be measured by MALDI-TOF (Matrix-Assisted Laser Desorption Ionization Time of Flight) mass spectrometry.

Examples of the PEG lipid include PEG-phospholipid, PEG-ceramide, PEG-diacylglycerol, and PEG-cholesterol. The PEG lipid is preferably diacylglycerol PEG with a PEG chain number-average molecular weight of 1,000 to 10,000, more preferably dimyristoylglycerol PEG with a PEG chain number-average molecular weight of 1,000 to 10,000 (particularly 1,2-dimyristoyl-rac-glycero-3-methylpolyoxyethylene, i.e., a compound in which methoxypolyethylene glycol chain is attached to 1,2-dimyristoyl-rac-glycerin) and/or distearoylglycerol PEG with a PEG chain number-average molecular weight of 1,000 to 10,000, further preferably dimyristoylglycerol PEG with a PEG chain number-average molecular weight of 1,000 to 10,000 (particularly 1,2-dimyristoyl-rac-glycero-3-methylpolyoxyethylene).

The concentration of PEG lipid in the alcohol solution is preferably 0.01 to 10 mM, more preferably 0.05 to 5 mM, from the aspect of lipid solubility.

The molar ratio of PEG lipid to ionic lipid (PEG lipid/ionic lipid) used in step (a) is preferably 0.001 to 0.5, more preferably 0.01 to 0.05, from the aspects of the stability of nucleic acid-encapsulating lipid nanoparticles and nucleic acid delivery efficiency.

Phospholipid

The alcohol solution used in step (a) may further contain a phospholipid. Only one kind of phospholipid may be used, or two or more kinds thereof may be used in combination.

Examples of phospholipids include 1,2-diacyl-sn-glycero-3-phosphocholine (PC), 1,2-diacyl-sn-glycero-3-phosphatidylethanolamine (PE), 1,2-diacyl-sn-glycero-3-phosphatidylserine (PS), 1,2-diacyl-sn-glycero-3-phosphatidylglycerol (PG), 1,2-diacyl-sn-glycero-3-phosphatidic acid (PA), and lyso forms thereof.

Specific examples of phospholipid include 1,2-didecanoyl-sn-glycero-3-phosphocholine (DDPC), 1,2-dilauroyl-sn-glycero-3-phosphocholine (DLPC), 1,2-dimyristoyl-sn-glycero-3-phosphocholine (DMPC), 1,2-dipalmitoyl-sn-glycero-3-phosphocholine (DPPC), 1,2-distearoyl-sn-glycero-3-phosphocholine (DSPC), 1,2-dioleoyl-sn-glycero-3-phosphocholine (DOPC), 1,2-dilinoleoyl-sn-glycero-3-phosphocholine (DLoPC), 1,2-dierucoyl-sn-glycero-3-phosphocholine (DEPC), 1-myristoyl-2-palmitoyl-sn-glycero-3-phosphocholine (MPPC), 1-myristoyl-2-stearoyl-sn-glycero-3-phosphocholine (MSPC), 1-palmitoyl-2-myristoyl-sn-glycero-3-phosphocholine (PMPC), 1-palmitoyl-2-stearoyl-sn-glycero-3-phosphocholine (PSPC), 1-palmitoyl-2-oleoyl-sn-glycero-3-phosphocholine (POPC), 1-stearoyl-2-oleoyl-sn-glycero-3-phosphocholine (SOPC).

The above-mentioned specific example of phospholipid, such as PC, may be appropriately converted to PE, PS, PG, or PA. In other words,

(i) the above-mentioned specific example of phospholipid, "sn-glycero-3-phosphocholine", can be converted to "sn-glycero-3-phosphatidylethanolamine" (e.g., 1,2-didecanoyl-sn-glycero-3-phosphatidylethanolamine),
(ii) the above-mentioned specific example of phospholipid, "sn-glycero-3-phosphocholine", can be converted to "sn-glycero-3-phosphatidylserine" (e.g., 1,2-didecanoyl-sn-glycero-3-phosphatidylserine),
(iii) the above-mentioned specific example of phospholipid, "sn-glycero-3-phosphocholine", can be converted to "sn-glycero-3-phosphatidylglycerol" (e.g., 1,2-didecanoyl-sn-glycero-3-phosphatidylglycerol), or
(iv) the above-mentioned specific example of phospholipid, "sn-glycero-3-phosphocholine", can be converted to "sn-glycero-3-phosphatidic acid" (e.g., 1,2-didecanoyl-sn-glycero-3-phosphatidic acid).

The phospholipid to be used in the present invention is preferably PC and/or PE, more preferably at least one

selected from the group consisting of DOPC, DSPC, DEPC, POPC, DOPE (1,2-dioleoyl-sn-glycero-3-phosphoethanolamine), and POPE (1-palmitoyl-2-oleoyl-sn-glycero-3-phosphoethanolamine), further preferably DOPC and/or DOPE, particularly preferably DOPC or DOPE.

When phospholipid is used in step (a), the concentration of the phospholipid in the alcohol solution is preferably 0.1 to 25 mM, more preferably 0.1 to 20 mM, from the aspect of lipid solubility.

When phospholipid is used in step (a), the molar ratio of phospholipid to ionic lipid (phospholipid/ionic lipid) is preferably 0.01 to 1.0, more preferably 0.1 to 0.9, from the aspects of the stability of the nucleic acid-encapsulating lipid nanoparticles and the nucleic acid delivery efficiency.

Alcohol

Examples of the alcohol to be used in the alcohol solution used in step (a) include ethanol and tert-butanol. Only one alcohol may be used, or two or more alcohols may be used in combination. From the aspects of lipid solubility and the production of nucleic acid-encapsulating lipid nanoparticles, the alcohol is preferably ethanol and/or tert-butanol, more preferably ethanol.

Citrate buffer

The pH of the citrate buffer used in step (a) is 3 to 6.5, preferably 3 to 6, more preferably 4 to 6, from the aspects of the particle size and nucleic acid encapsulation rate of the nucleic acid-encapsulating lipid nanoparticles.

The citrate buffer contains citric acid and a salt thereof (specifically, trisodium citrate). From the aspect of nucleic acid stability, the total concentration of citric acid and a salt thereof in the citrate buffer used in step (a) is preferably 10 to 100 mM, more preferably 10 to 80 mM.

Nucleic acid

Examples of the nucleic acid to be used in the present invention include, but are not limited to, DNA, RNA, chimera nucleic acid of RNA, DNA/RNA hybrid and the like. While any nucleic acid having 1 to 3 chains can be used, it is preferably a single strand or double strand. The nucleic acid may be other type of nucleotide such as N-glycoside of purine or pyrimidine base or other oligomer having a non-nucleotide backbone (e.g., commercially available peptide nucleic acid (PNA), etc.), other oligomer containing a special bond (said oligomer comprising base pairing or a nucleotide having a configuration permitting attachment of base, which are found in DNA and RNA) and the like. Furthermore, for example, it may be a nucleic acid added with known modification, a nucleic acid with a label known in the field, a nucleic acid with a cap, a methylated nucleic acid, one or more natural nucleotides substituted by an analog, a nucleic acid with intramolecular nucleotidyl modification, a nucleic acid with non-charge bond (e.g., methylsulfonate, phosphotriester, phosphoramidate, carbamate and the like), a nucleic acid with a charged bond or sulfur-containing bond (e.g., phosphorothioate, phosphorodithioate and the like), for example, a nucleic acid with a side chain group such as protein (e.g., nuclease, nuclease inhibitor, toxin, antibody, signal peptide, poly-L-lysine and the like), sugar (e.g., monosaccharide and the like) and the like, a nucleic acid with an intercalating compound (e.g., acridine, psoralen and the like), a nucleic acid with a chelate compound (e.g., metal, radioactive metal, boron, oxidative metal and the like), a nucleic acid containing an alkylating agent, or a nucleic acid with a modified bond (e.g., $\alpha$ anomer-type nucleic acid and the like).

The type of the DNA to be used in the present invention is not particularly limited, and can be selected as appropriate according to the purpose of use. For example, plasmid DNA, cDNA, antisense DNA, chromosomal DNA, PAC, BAC, CpG oligosaccharide, and the like can be mentioned. Preferred are plasmid DNA, cDNA and antisense DNA, and more preferred is plasmid DNA. A circular DNA such as plasmid DNA and the like can be digested as appropriate with a restriction enzyme and the like, and also used as a linear DNA.

The type of the RNA to be used in the present invention is not particularly limited, and can be selected as appropriate according to the purpose of use. For example, siRNA, miRNA, shRNA, antisense RNA, messenger RNA (mRNA), single strand RNA genome, double strand RNA genome, RNA replicon, transfer RNA, ribosomal RNA and the like can be mentioned, with preference given to siRNA, miRNA, shRNA, mRNA, antisense RNA, and RNA replicon.

The nucleic acid to be used in the present invention is preferably purified by a method generally used by those of ordinary skill in the art.

The preferred concentration of nucleic acid in the citrate buffer used in step (a) is determined based on the total lipid concentration in the alcohol. From this aspect, the concentration of nucleic acid in the citrate buffer is preferably 0.5 to 400 $\mu$g/mL, more preferably 1 to 200 $\mu$g/mL.

When the molar ratio of the total amino groups in the ionic lipid to phosphate groups of the nucleic acid (total amino groups of ionic lipid/phosphate groups of nucleic acid) (sometimes referred to as the "N/P ratio" in the present specification) used in step (a) is low, the nucleic acid encapsulation efficiency and gene delivery efficiency may decrease.

Therefore, the N/P ratio is preferably not less than 7. On the other hand, when the N/P ratio is high, toxicity derived from the ionic lipid may be expressed. Therefore, the N/P ratio is more preferably 7 to 130, further preferably 17 to 70.

In step (a) of the present invention, a suspension of nucleic acid-encapsulating lipid nanoparticles is prepared by mixing an alcohol solution containing an ionic lipid having a tertiary amino group, a sterol, and a PEG lipid (hereinafter sometimes referred to as the "lipid alcohol solution") with a citrate buffer having a pH of 3 to 6.5 in which nucleic acid is dispersed (hereinafter sometimes referred to as the "nucleic acid-containing buffer"). In addition, in the nucleic acid-containing buffer, the nucleic acid may or may not be dissolved in the buffer. That is, the nucleic acid-containing buffer may be a nucleic acid solution in which the nucleic acid is dispersed in a dissolved state, or a dispersion in which the nucleic acid is dispersed without dissolving.

The mixing in step (a) is preferably performed using a device including a micro flow path or a vortex, more preferably using a device including a micro flow path. Examples of the device including micro flow path include the NanoAssemblr (registered trademark) ultra high-speed nanomedicament producing apparatus (Precision NanoSystems), Fully Automated Library Synthesis System ALiS (Particle Works), and ANP System (Particle Works).

When mixing the nucleic acid-containing buffer and the lipid alcohol solution, the volume ratio (nucleic acid-containing buffer:lipid alcohol solution) is preferably 1:1 to 20:1, more preferably 2:1 to 12:1, from the aspects of the particle size and nucleic acid encapsulation rate of the nucleic acid-encapsulating lipid nanoparticles. When mixing the nucleic acid-containing buffer and the lipid alcohol solution in a device including micro flow path, the total flow rate of the nucleic acid-containing buffer and the lipid alcohol solution is preferably 1 to 20 mL/min, more preferably 10 to 15 mL/min, from the aspect of the particle size of the nucleic acid-encapsulating lipid nanoparticles. Here, the "total flow rate of the nucleic acid-containing buffer and the lipid alcohol solution" refers to the sum of the flow rate of the nucleic acid-containing buffer and the flow rate of the lipid alcohol solution. For example, when the volume ratio of nucleic acid-containing buffer to lipid alcohol solution is 3:1 and the total flow rate is 4 mL/min, the flow rate of the nucleic acid-containing buffer is 3 mL/min, and the flow rate of the lipid alcohol solution is 1 mL/min.

The temperature at which the nucleic acid-containing buffer and the lipid alcohol solution are mixed is preferably 0 to 50°C, more preferably 10 to 30°C, from the aspect of maintaining a constant lipid state during formation of nucleic acid-encapsulating lipid nanoparticles and preparing nucleic acid-encapsulating lipid nanoparticles with high reproducibility.

When using a device including a micro flow path, the cross-sectional shape of the micro flow path may be either circular or rectangular. When using a device including a rectangular micro flow path with a rectangular cross-section, the vertical and horizontal lengths which are inner diameters of the rectangular micro flow path are each independently preferably 50 to 700 μm, more preferably 70 to 500 μm, from the aspect of the particle size and nucleic acid encapsulation rate of the nucleic acid-encapsulating lipid nanoparticles.

The concentration of nucleic acid in the suspension of nucleic acid-encapsulating lipid nanoparticles obtained in step (a) is preferably 0.01 to 100 μg/mL, more preferably 0.05 to 50 μg/mL.

Step (b)

In step (b) of the present invention, the suspension of nucleic acid-encapsulating lipid nanoparticles is concentrated by ultrafiltration and diluted with a Tris buffer having pH 5.2 to 9.0, whereby the dispersion medium of the aforementioned suspension is exchanged with the aforementioned Tris buffer.

The aforementioned dilution and the aforementioned concentration in step (b) may each be performed once or multiple times. The aforementioned dilution and the aforementioned concentration are preferably each performed multiple times.

Either aforementioned dilution or the aforementioned concentration in step (b) can be performed first. In other words, (i) the suspension of nucleic acid-encapsulating lipid nanoparticles may be diluted by adding the aforementioned Tris buffer, and then concentrated using ultrafiltration, or (ii) the suspension of nucleic acid-encapsulating lipid nanoparticles may be concentrated by ultrafiltration, and the obtained concentrate may be diluted by adding the aforementioned Tris buffer. However, after the aforementioned concentration, the aforementioned dilution is performed to exchange the dispersion medium of the suspension of nucleic acid-encapsulating lipid nanoparticles with the aforementioned Tris buffer, whereby a suspension of nucleic acid-encapsulating lipid nanoparticles with good storage stability (i.e., the aforementioned Tris buffer in which nucleic acid-encapsulating lipid nanoparticles are dispersed) is produced.

The ultrafiltration in step (b) is preferably centrifugal ultrafiltration. As used herein, the "centrifugal ultrafiltration" refers to ultrafiltration that utilizes centrifugal force.

Between step (a) and step (b), step (c) may be performed in which the suspension of nucleic acid-encapsulating lipid nanoparticles obtained in step (a) is concentrated by ultrafiltration and diluted with a dispersion medium other than the aforementioned Tris buffer (e.g., PBS) to exchange the dispersion medium of the aforementioned suspension with a dispersion medium other than the aforementioned Tris buffer (e.g., PBS). In this case, the dispersion medium of the suspension of nucleic acid-encapsulating lipid nanoparticles obtained in step (c) is exchanged with the aforementioned Tris buffer in step (b). Similar to step (b), the aforementioned dilution and the aforementioned concentration in step (c) may

each be performed once or multiple times. Similar to step (b), moreover, the aforementioned dilution and the aforementioned concentration in step (c) may be performed in any order. For ease of operation, it is preferable not to perform step (c). In other words, in step (b), the dispersion medium of the suspension of nucleic acid-encapsulating lipid nanoparticles obtained in step (a) is preferably exchanged with the aforementioned Tris buffer.

Tris buffer

The pH of the Tris buffer used in step (b) is 5.2 to 9.0, more preferably 5.5 to 8.5, further preferably 6.0 to 8.0, from the aspects of particle size and nucleic acid encapsulation rate. The Tris buffer may contain sodium chloride. When sodium chloride is contained, the concentration thereof in the Tris buffer is preferably 10 to 154 mM, more preferably 30 to 154 mM.

Examples of the Tris buffer include Tris-buffered saline (TBS), Tris-HCl buffer, TE buffer, TAE buffer, and TBE buffer and the like. Tris-buffered saline (TBS) contains tris-hydroxymethylaminomethane and hydrochloride thereof, and sodium chloride. Tris-HCl buffer contains tris-hydroxymethylaminomethane and hydrochloride thereof. TE buffer contains tris-hydroxymethylaminomethane and hydrochloride thereof, and ethylenediaminetetraacetic acid. TAE buffer contains trishydroxymethylaminomethane and acetate thereof, and ethylenediaminetetraacetic acid. TBE buffer contains trishydroxymethylaminomethane and borate thereof, and ethylenediaminetetraacetic acid. Tris buffer is preferably Tris-buffered saline (TBS).

From the aspect of nucleic acid encapsulation rate, the total concentration of trishydroxymethylaminomethane and a salt thereof in the Tris buffer used in step (b) is preferably 10 to 200 mM, more preferably 10 to 100 mM.

When Tris-buffered saline (TBS) is used in step (b), the concentration of sodium chloride in TBS is preferably 140 to 160 mM, from the aspect of nucleic acid stability.

The concentration of nucleic acid in the suspension of nucleic acid-encapsulating lipid nanoparticles obtained in step (b) is preferably 0.01 to 100 μg/mL, more preferably 0.05 to 50 μg/mL.

Nucleic acid-encapsulating lipid nanoparticles

The nucleic acid encapsulation rate of the nucleic acid-encapsulating lipid nanoparticles produced by the method of the present invention is preferably 45% or more. The nucleic acid encapsulation rate can be measured using Ribogreen (registered trademark) assay.

The particle size of the nucleic acid-encapsulating lipid nanoparticles produced by the method of the present invention is not particularly limited, and is preferably 10 nm to 500 nm, more preferably 30 nm to 300 nm, further preferably 50 to 120 nm. The particle size can be measured by using a particle size distribution measuring device such as Zetasizer Nano (Malvern) or the like. In the present specification, the "particle size" means an average particle size (number average) measured by a dynamic light scattering method.

The surface potential (zeta potential) of the nucleic acid-encapsulating lipid nanoparticles produced by the method of the present invention is not particularly limited and preferably -15 to +15 mV, further preferably -10 to +10 mV. In conventional gene transfer, particles with positively charged surface potential have been mainly used. This is useful as a method for promoting electrostatic interactions with heparin sulfate on the negatively-charged cell surface to enhance uptake into cells. However, a positive surface potential can result in inhibition of release of nucleic acid from the carrier through interaction with the delivered nucleic acid within cells, and inhibition of protein synthesis through interaction between mRNA and the delivered nucleic acid. This problem can be solved by adjusting the surface potential to fall within the above-mentioned range. The surface potential can be measured using a zeta potential measuring apparatus such as Zetasizer Nano and the like. The surface potential of the lipid nanoparticles can be adjusted by the composition of the constituent component of the lipid nanoparticles.

The nucleic acid-encapsulating lipid nanoparticles produced by the method of the present invention can be administered in vivo for the purpose of, for example, prevention and/or treatment of diseases. Therefore, the nucleic acid to be used in the present invention is preferably one having a preventive and/or therapeutic activity against a given disease (prophylactic/therapeutic nucleic acid). Examples of such nucleic acid include nucleic acids and the like used for so-called gene therapy.

Storage of nucleic acid-encapsulating lipid nanoparticles

The storage temperature of nucleic acid-encapsulating lipid nanoparticles produced by the method of the present invention is preferably 0°C to 50°C, more preferably 0°C to 30°C, further preferably 0°C to 20°C, particularly preferably 0°C to 10°C, most preferably 0°C to 5°C.

The nucleic acid-encapsulating lipid nanoparticles produced by the method of the present invention are preferably stored in a refrigerator. In other words, the method of the present invention is preferably a method for producing nucleic acid-encapsulating lipid nanoparticles for refrigerated storage. As used herein, the "nucleic acid-encapsulating

lipid nanoparticles for refrigerated storage" mean nucleic acid-encapsulating lipid nanoparticles stored at 0°C to 20°C. The storage temperature of the nucleic acid-encapsulating lipid nanoparticles for refrigerated storage is preferably 0°C to 10°C, more preferably 0°C to 5°C.

The storage stability of nucleic acid-encapsulating lipid nanoparticles can be evaluated by measuring the particle size, polydispersity index (PdI), nucleic acid (e.g., mRNA) encapsulation rate, and in vitro or in vivo nucleic acid introduction efficiency of the lipid nanoparticles before and after storage. When evaluating in vitro or in vivo nucleic acid introduction efficiency, it is preferable that the rate of decrease in nucleic acid introduction efficiency before and after storage is 30% or less when stored at a storage temperature of 0°C to 50°C for 7 days, more preferable that the rate of decrease in nucleic acid introduction efficiency before and after storage is 20% or less when stored at a storage temperature of 0°C to 5°C for 30 days.

Method for introducing nucleic acid into cell

The present invention also provides

(i) a method for introducing a nucleic acid into a cell, including contacting nucleic acid-encapsulating lipid nanoparticles produced by the aforementioned method with the aforementioned cell in vitro, and
(ii) a method for introducing a nucleic acid into a target cell of a living organism, comprising administering nucleic acid-encapsulating lipid nanoparticles produced by the aforementioned method to the aforementioned living organism.

The contacting the nucleic acid-encapsulating lipid nanoparticles with the cell in vitro is specifically explained below.

The cells are suspended in a suitable medium several days before contact with the nucleic acid-encapsulating lipid nanoparticles, and cultured under appropriate conditions. At the time of contact with the nucleic acid-encapsulating lipid nanoparticles, the cells may or may not be in a proliferative phase.

The culture medium on contact may be a serum-containing medium or a serum-free medium, wherein the serum concentration of the medium is preferably not more than 30 wt%, more preferably not more than 20 wt%, since when the medium contains excess protein such as serum and the like, the contact between the nucleic acid-encapsulating lipid nanoparticles and the cell may be inhibited.

The cell density on contact is not particularly limited, and can be appropriately determined in consideration of the kind of the cell and the like. It is generally within the range of $1\times10^4$ to $1\times10^7$ cells/mL.

A suspension of nucleic acid-encapsulating lipid nanoparticles is added to the above-mentioned cells. The amount of the suspension to be added is not particularly limited, and can be appropriately determined in consideration of the cell number and the like. The concentration of the nucleic acid-encapsulating lipid nanoparticles to be contacted with the cells is not particularly limited as long as the desired introduction of the nucleic acid into the cells can be achieved. The lipid concentration is generally 1 to 300 nmol/mL, preferably 10 to 200 nmol/mL, and the concentration of the nucleic acid is generally 0.01 to 100 $\mu$g/mL, preferably 0.05 to 10 $\mu$g/mL.

After the aforementioned suspension is added to cells, the cells are cultured. The temperature, humidity and $CO_2$ concentration during culturing are appropriately determined in consideration of the kind of the cell. When the cell is derived from a mammal, generally, the temperature is about 37°C, humidity is about 95% and $CO_2$ concentration is about 5%. While the culture period can also be appropriately determined in consideration of the conditions such as the kind of the cell and the like, it is generally 0.1 to 96 hr, preferably 0.2 to 72 hr, more preferably 0.5 to 48 hr. When the above-mentioned culture time is too short, the nucleic acid is not sufficiently introduced into the cells, and when the culture time is too long, the cells may become weak.

By the above-mentioned culture, the nucleic acid is introduced into cells. The culture is further continued preferably by exchanging the medium with a fresh medium, or adding a fresh medium to the medium. When the cell is a mammal-derived cell, the fresh medium preferably contains a serum or nutrition factor.

As mentioned above, a nucleic acid can be introduced into the target cells of a living organism not only outside the body (in vitro) but also in the body (in vivo) by using nucleic acid-encapsulating lipid nanoparticles. That is, by administration of the nucleic acid-encapsulating lipid nanoparticles to a living organism, the nucleic acid-encapsulating lipid nanoparticles reach and contact with the target cells, and the nucleic acid in the lipid nanoparticles is introduced into the cells in vivo. The subject to which the nucleic acid-encapsulating lipid nanoparticles can be administered is not particularly limited and, for example, vertebrates such as mammals (e.g., human, monkey, mouse, rat, hamster, bovine, etc.), birds (e.g., chicken, ostrich, etc.), amphibia (e.g., frog, etc.), fishes (e.g., zebrafish, rice-fish, etc.) and the like, invertebrates such as insects (e.g., silk moth, moth, Drosophila, etc.) and the like, plants and the like can be mentioned. The subject of administration of the nucleic acid-encapsulating lipid nanoparticles is preferably human or other mammal.

The kind of the target cell is not particularly limited, and a nucleic acid can be introduced into cells in various tissues (e.g., liver, kidney, pancreas, lung, spleen, heart, blood, muscle, bone, brain, stomach, small intestine, large

intestine, skin, adipose tissue, lymph node, tumor, etc.) by using the nucleic acid-encapsulating lipid nanoparticles.

The administration method of the nucleic acid-encapsulating lipid nanoparticles to a subject of administration (e.g., vertebrate, invertebrate and the like) is not particularly limited as long as the lipid nanoparticles reaches and contacts with the target cells, and the compound introduced into the lipid nanoparticles can be introduced into the cell, and an administration method known per se (e.g., oral administration, parenteral administration (e.g., intravenous administration, intramuscular administration, topical administration, transdermal administration, subcutaneous administration, intraperitoneal administration, spray, etc.), etc.) can be appropriately selected in consideration of the kind and the site of the target cell and the like. The dose of the nucleic acid-encapsulating lipid nanoparticles is not particularly limited as long as the introduction of the nucleic acid into the cells can be achieved, and can be appropriately selected in consideration of the kind of the subject of administration, administration method, the kind of the compound to be introduced, the kind and the site of the target cell and the like.

Method for producing pharmaceutical composition

The nucleic acid-encapsulating lipid nanoparticles produced by the method of the present invention can be used as a drug delivery system for selectively delivering a nucleic acid and the like into a particular cell, and is useful for, for example, DNA vaccines by introducing antigen gene into dendritic cells, gene therapy drugs for tumor, nucleic acid pharmaceutical products that suppress expression of target genes by utilizing RNA interference, and the like. Therefore, the present invention also provides a production method of a pharmaceutical composition, including the aforementioned method.

Nucleic acid-introducing agent

When the nucleic acid-encapsulating lipid nanoparticles produced by the method of the present invention are used as a nucleic acid-introducing agent, the nucleic acid-encapsulating lipid nanoparticles can be formulated according to a conventional method.

When the nucleic acid-introducing agent is provided as a reagent for studies, the nucleic acid-encapsulating lipid nanoparticles may be provided as they are as the nucleic acid-introducing agent, or the nucleic acid-introducing agent of the present invention may be provided as a sterile solution or suspension of nucleic acid-encapsulating lipid nanoparticles with, for example, water or other physiologically acceptable liquid (e.g., water-soluble solvent (e.g., malic acid buffer, etc.), organic solvent (e.g., ethanol, methanol, DMSO, tert-butanol, etc.), or a mixture of aqueous solvent and organic solvent, etc.). The nucleic acid-introducing agent may appropriately contain physiologically acceptable additive (e.g., excipient, vehicle, preservative, stabilizer, binder, etc.), which are known per se.

Furthermore, when the nucleic acid-introducing agent is provided as a medicament, the nucleic acid-encapsulating lipid nanoparticles can be used as they are, or the nucleic acid-encapsulating lipid nanoparticles can be used together with known pharmaceutically acceptable additives (e.g., carrier, flavor, excipient, vehicle, preservative, stabilizer, binder, etc.) and mixed in a unit dosage form required for generally accepted pharmaceutical practice, to produce an oral agent (e.g., tablet, capsule, etc.) or parenteral agent (e.g., injectable preparation, spray, etc.), preferably parenteral agent (more preferably, injectable preparation).

The nucleic acid-introducing agent can be formulated into a preparation not only for adults but also for children.

[Example]

The present invention is described in more detail in the following by referring to Examples; however, the present invention is not limited to the following Examples and the like.

In the following Examples, the ionic lipids represented by the formula (1) are shown by the names listed in the aforementioned Tables. Nucleic acid-encapsulating lipid nanoparticles may be abbreviated as "nucleic acid-encapsulating nanoparticles". The abbreviations used in the following Examples mean the following.

Chol: cholesterol
DMG-PEG2000: 1,2-dimyristoyl-rac-glycero-3-methylpolyoxyethylene (number average molecular weight of PEG chain: 2000)
DOPC: 1,2-dioleoyl-sn-glycero-3-phosphocholine
DOPE: 1,2-dioleoyl-sn-glycero-3-phosphoethanolamine
FBS: fetal bovine serum
LNP: lipid nanoparticle
MES: 2-morpholinoethanesulfonic acid
N/P ratio: molar ratio of total amino groups of ionic lipid to phosphate groups of nucleic acid (total amino groups of ionic

lipid/phosphate groups of nucleic acid)
PBS: phosphate-buffered saline
TBS: Tris-buffered saline
Tris: tris(hydroxymethyl)aminomethane

Example 1

As ionic lipid, SS-OP was used. The lipid composition was SS-OP:DOPC:Chol:DMG-PEG2000=52.5:7.5:40:1.5 in molar ratio.
The concentrations of each lipid in the ethanol solution used were as follows.

SS-OP: 1.8 mM
DOPC: 0.3 mM
Chol: 1.3 mM
DMG-PEG2000: 0.05 mM

1200 μL of mRNA-containing buffer solution (mRNA concentration: 5 μg/mL) in which mRNA was dispersed in citrate buffer (total concentration of citric acid and salt thereof: 20 mM, pH: 5.0) and 450 μL of lipid ethanol solution were weighed into a syringe. Using a NanoAssmblr (registered trademark) ultrafast nanomedicine production device (manufactured by Precision NanoSystems), the mRNA-containing buffer solution and the lipid ethanol solution were mixed under the conditions of flow rate (volume ratio) of mRNA-containing buffer solution:lipid ethanol solution of 3:1, total flow rate: 12 mL/min, inner diameter of micro flow path: 130×300 μm (square), and syringe holder temperature: 25°C to prepare a suspension of nucleic acid-encapsulating nanoparticles (N/P ratio: 68, nucleic acid concentration in suspension: 3.7 μg/mL).
After adding 1000 μL of TBS (total concentration of Tris and salt thereof: 50 mM, NaCl concentration: 150 mM, pH: 7.4) to the recovered suspension, the obtained mixture was transferred to Amicon Ultra 4 and concentrated to about 500 μL by ultrafiltration under centrifugation conditions (25°C, 1000 g, 3 min).
The obtained concentrate was diluted to 4 mL with the aforementioned TBS and then concentrated again to about 200 μL by ultrafiltration under centrifugation conditions (25°C, 1000 g, 3 min). This dilution and concentration operation was repeated two more times, after which 0.2 mL of the aforementioned TBS was added to the concentrate to obtain a suspension of nucleic acid-encapsulating nanoparticles (nucleic acid concentration in the suspension: 10 μg/mL).

Comparative Example 1

As ionic lipid, SS-OP was used. The lipid composition was SS-OP:DOPC:Chol:DMG-PEG2000=52.5:7.5:40:1.5 in molar ratio.
The concentrations of each lipid in the lipid ethanol solution used were as follows.

SS-OP: 1.8 mM
DOPC: 0.3 mM
Chol: 1.3 mM
DMG-PEG2000: 0.05 mM

1200 μL of mRNA-containing buffer solution (mRNA concentration: 5 μg/mL) in which mRNA was dispersed in malic acid buffer (total concentration of malic acid and salt thereof: 20 mM, pH: 3.0) and 450 μL of lipid ethanol solution were weighed into a syringe. Using a NanoAssmblr (registered trademark) ultrafast nanomedicine production device (manufactured by Precision NanoSystems), the mRNA-containing buffer solution and the lipid ethanol solution were mixed under the conditions of flow rate of mRNA-containing buffer solution:lipid ethanol solution of 3:1, total flow rate: 4 mL/min, inner diameter of micro flow path: 130×300 μm (square), and syringe holder temperature: 25°C to prepare a suspension of nucleic acid-encapsulating nanoparticles (N/P ratio: 68, nucleic acid concentration in suspension: 3.7 μg/mL).
After adding 1000 μL of MES buffer (total concentration of MES and salt thereof: 20 mM, pH: 6.5) to the recovered suspension, the obtained mixture was transferred to Amicon Ultra 4 and concentrated to about 500 μL by ultrafiltration under centrifugation conditions (25°C, 1000 g, 3 min).
The obtained concentrate was diluted to 4 mL with PBS (pH:7.4) and then concentrated again to about 200 μL by ultrafiltration under centrifugation conditions (25°C, 1000 g, 3 min). This dilution and concentration operation was repeated two more times, after which 0.2 mL of the aforementioned PBS was added to the concentrate to obtain a suspension of nucleic acid-encapsulating nanoparticles (nucleic acid concentration in the suspension: 10 μg/mL).

Comparative Example 2

As ionic lipid, SS-OP was used. The lipid composition was SS-OP:DOPC:Chol:DMG-PEG2000=52.5:7.5:40:1.5 in molar ratio.
The concentrations of each lipid in the lipid ethanol solution used were as follows.

SS-OP: 1.8 mM
DOPC: 0.3 mM
Chol: 1.3 mM
DMG-PEG2000: 0.05 mM

1200 μL of mRNA-containing buffer solution (mRNA concentration: 5 μg/mL) in which mRNA was dispersed in malic acid buffer (total concentration of malic acid and salt thereof: 20 mM, pH: 5.0) and 450 μL of lipid ethanol solution were weighed into a syringe. Using a NanoAssmblr (registered trademark) ultrafast nanomedicine production device (manufactured by Precision NanoSystems), the mRNA-containing buffer solution and the lipid ethanol solution were mixed under the conditions of flow rate of mRNA-containing buffer solution:lipid ethanol solution of 3:1, total flow rate: 4 mL/min, inner diameter of micro flow path: 130×300 μm (square), and syringe holder temperature: 25°C to prepare a suspension of nucleic acid-encapsulating nanoparticles (N/P ratio: 68, nucleic acid concentration in suspension: 3.7 μg/mL).
After adding 1000 μL of MES buffer (total concentration of MES and salt thereof: 20 mM, pH: 6.5) to the recovered suspension, the obtained mixture was transferred to Amicon Ultra 4 and concentrated to about 500 μL by ultrafiltration under centrifugation conditions (25°C, 1000 g, 3 min).
The obtained concentrate was diluted to 4 mL with PBS (pH:7.4) and then concentrated again to about 200 μL by ultrafiltration under centrifugation conditions (25°C, 1000 g, 3 min). This dilution and concentration operation was repeated two more times, after which 0.2 mL of the aforementioned PBS was added to the concentrate to obtain a suspension of nucleic acid-encapsulating nanoparticles (nucleic acid concentration in the suspension: 10 μg/mL).

Comparative Example 3

As ionic lipid, SS-OP was used. The lipid composition was SS-OP:DOPC:Chol:DMG-PEG2000=52.5:7.5:40:1.5 in molar ratio.
The concentrations of each lipid in the lipid ethanol solution used were as follows.

SS-OP: 1.8 mM
DOPC: 0.3 mM
Chol: 1.3 mM
DMG-PEG2000: 0.05 mM

1200 μL of mRNA-containing buffer solution (mRNA concentration: 5 μg/mL) in which mRNA was dispersed in malic acid buffer (total concentration of malic acid and salt thereof: 20 mM, pH: 3.0) and 450 μL of lipid ethanol solution were weighed into a syringe. Using a NanoAssmblr (registered trademark) ultrafast nanomedicine production device (manufactured by Precision NanoSystems), the mRNA-containing buffer solution and the lipid ethanol solution were mixed under the conditions of flow rate of mRNA-containing buffer solution:lipid ethanol solution of 3:1, total flow rate: 4 mL/min, inner diameter of micro flow path: 130×300 μm (square), and syringe holder temperature: 25°C to prepare a suspension of nucleic acid-encapsulating nanoparticles (N/P ratio: 200, nucleic acid concentration in suspension: 3.7 μg/mL).
After adding 1000 μL of MES buffer (total concentration of MES and salt thereof: 20 mM, pH: 6.5) to the recovered suspension, the obtained mixture was transferred to Amicon Ultra 4 and concentrated to about 500 μL by ultrafiltration under centrifugation conditions (25°C, 1000 g, 3 min).
The obtained concentrate was diluted to 4 mL with TBS (total concentration of Tris and salt thereof: 50 mM, NaCl concentration: 150 mM, pH:7.4) and then concentrated again to about 200 μL by ultrafiltration under centrifugation conditions (25°C, 1000 g, 3 min). This dilution and concentration operation was repeated two more times, after which 0.2 mL of the aforementioned TBS was added to the concentrate to obtain a suspension of nucleic acid-encapsulating nanoparticles (nucleic acid concentration in the suspension: 10 μg/mL).

Comparative Example 4

As ionic lipid, SS-OP was used. The lipid composition was SS-OP:DOPC:Chol:DMG-PEG2000=52.5:7.5:40:1.5

in molar ratio.

The concentrations of each lipid in the lipid ethanol solution used were as follows.

SS-OP: 1.8 mM
DOPC: 0.3 mM
Chol: 1.3 mM
DMG-PEG2000: 0.05 mM

1200 μL of mRNA-containing buffer solution (mRNA concentration: 5 μg/mL) in which mRNA was dispersed in citrate buffer (total concentration of citric acid and salt thereof: 20 mM, pH: 3.0) and 450 μL of lipid ethanol solution were weighed into a syringe. Using a NanoAssmblr (registered trademark) ultrafast nanomedicine production device (manufactured by Precision NanoSystems), the mRNA-containing buffer solution and the lipid ethanol solution were mixed under the conditions of flow rate of mRNA-containing buffer solution:lipid ethanol solution of 3:1, total flow rate: 4 mL/min, inner diameter of micro flow path: 130×300 μm (square), and syringe holder temperature: 25°C to prepare a suspension of nucleic acid-encapsulating nanoparticles (N/P ratio: 68, nucleic acid concentration in suspension: 3.7 μg/mL).

After adding 1000 μL of MES buffer (total concentration of MES and salt thereof: 20 mM, pH: 6.5) to the recovered suspension, the obtained mixture was transferred to Amicon Ultra 4 and concentrated to about 500 μL by ultrafiltration under centrifugation conditions (25°C, 1000 g, 3 min).

The obtained concentrate was diluted to 4 mL with PBS (pH:7.4) and then concentrated again to about 200 μL by ultrafiltration under centrifugation conditions (25°C, 1000 g, 3 min). This dilution and concentration operation was repeated two more times, after which 0.2 mL of the aforementioned PBS was added to the concentrate to obtain a suspension of nucleic acid-encapsulating nanoparticles (nucleic acid concentration in the suspension: 10 μg/mL).

Comparative Example 5

As ionic lipid, SS-OP was used. The lipid composition was SS-OP:DOPC:Chol:DMG-PEG2000=52.5:7.5:40:1.5 in molar ratio.

The concentrations of each lipid in the lipid ethanol solution used were as follows.

SS-OP: 1.8 mM
DOPC: 0.3 mM
Chol: 1.3 mM
DMG-PEG2000: 0.05 mM

1200 μL of mRNA-containing buffer solution (mRNA concentration: 5 μg/mL) in which mRNA was dispersed in citrate buffer (total concentration of citric acid and salt thereof: 20 mM, pH: 5.0) and 450 μL of lipid ethanol solution were weighed into a syringe. Using a NanoAssmblr (registered trademark) ultrafast nanomedicine production device (manufactured by Precision NanoSystems), the mRNA-containing buffer solution and the lipid ethanol solution were mixed under the conditions of flow rate of mRNA-containing buffer solution:lipid ethanol solution of 3:1, total flow rate: 4 mL/min, inner diameter of micro flow path: 130×300 μm (square), and syringe holder temperature: 25°C to prepare a suspension of nucleic acid-encapsulating nanoparticles (N/P ratio: 68, nucleic acid concentration in suspension: 3.7 μg/mL).

After adding 1000 μL of MES buffer (20 mM, pH: 6.5) to the recovered suspension, the obtained mixture was transferred to Amicon Ultra 4 and concentrated to about 500 μL by ultrafiltration under centrifugation conditions (25°C, 1000 g, 3 min).

The obtained concentrate was diluted to 4 mL with PBS (pH:7.4) and then concentrated again to about 200 μL by ultrafiltration under centrifugation conditions (25°C, 1000 g, 3 min). This dilution and concentration operation was repeated two more times, after which 0.2 mL of the aforementioned PBS was added to the concentrate to obtain a suspension of nucleic acid-encapsulating nanoparticles (nucleic acid concentration in the suspension: 10 μg/mL).

Comparative Example 6

As ionic lipid, SS-OP was used. The lipid composition was SS-OP:DOPC:Chol:DMG-PEG2000=52.5:7.5:40:1.5 in molar ratio.

The concentrations of each lipid in the lipid ethanol solution used were as follows.

SS-OP: 1.8 mM
DOPC: 0.3 mM
Chol: 1.3 mM

DMG-PEG2000: 0.05 mM

1200 μL of mRNA-containing buffer solution (mRNA concentration: 5 μg/mL) in which mRNA was dispersed in citrate buffer (20 mM, pH: 5.0) and 450 μL of lipid ethanol solution were weighed into a syringe. Using a NanoAssmblr (registered trademark) ultrafast nanomedicine production device (manufactured by Precision NanoSystems), the mRNA-containing buffer solution and the lipid ethanol solution were mixed under the conditions of flow rate of mRNA-containing buffer solution:lipid ethanol solution of 3:1, total flow rate: 4 mL/min, inner diameter of micro flow path: 130×300 μm (square), and syringe holder temperature: 25°C to prepare a suspension of nucleic acid-encapsulating nanoparticles (N/P ratio: 68, nucleic acid concentration in suspension: 3.7 μg/mL).

After adding 1000 μL of PBS (pH: 7.4) to the recovered suspension, the obtained mixture was transferred to Amicon Ultra 4 and concentrated to about 500 μL by ultrafiltration under centrifugation conditions (25°C, 1000 g, 3 min).

The obtained concentrate was diluted to 4 mL with PBS and then concentrated again to about 500 μL by ultrafiltration under centrifugation conditions (25°C, 1000 g, 3 min). The above operation was performed twice to obtain nucleic acid-encapsulating nanoparticles (nucleic acid concentration in the suspension: 10 μg/mL).

<u>Measurement of particle size, etc.</u>

The particle size, Polydispersity Index (PdI), and mRNA encapsulation rate of the nucleic acid-encapsulating nanoparticles obtained in Example 1 and Comparative Examples 1 to 6 were analyzed. The particle size and PdI were measured by the dynamic light scattering method using a Zetasizer (registered trademark). The mRNA encapsulation rate was measured by the Ribogreen (registered trademark) assay. The results are shown in Table 2. The particle size is shown as Z-Ave (Z-average) and Number Mean (number average) (same in the following). Since PdI varies depending on the type of average particle size, the PdI is shown to the right of Z-Ave and Number Mean in Table 2 (the same applies below).

[Table 2]

| | Z-Ave (nm) | PdI | Number Mean (nm) | PdI | mRNA encapsulation rate (%) |
|---|---|---|---|---|---|
| Example 1 | 97 | 0.096 | 68 | 0.078 | 83 |
| Comparative Example 1 | 94 | 0.138 | 63 | 0.081 | 83 |
| Comparative Example 2 | 94 | 0.101 | 63 | 0.084 | 84 |
| Comparative Example 3 | 94 | 0.121 | 67 | 0.072 | 82 |
| Comparative Example 4 | 123 | 0.110 | 84 | 0.101 | 89 |
| Comparative Example 5 | 92 | 0.098 | 68 | 0.071 | 86 |
| Comparative Example 6 | 92 | 0.098 | 61 | 0.085 | 84 |

As shown in Table 2, mRNA was encapsulated in LNP in Example 1, in which citrate buffer and TBS were used, and no difference in the mRNA encapsulation rate was observed between Example 1 and Comparative Examples 1 to 6, which used other conditions.

<u>Evaluation of nucleic acid introduction efficiency</u>

The nucleic acid-encapsulating nanoparticles obtained in Example 1 and Comparative Examples 1 to 6 were applied to HeLa cells to evaluate the nucleic acid introduction efficiency. In detail, HeLa cells were seeded in D-MEM medium (high glucose) (containing L-glutamine and phenol red) (10 v/v% FBS, 1 v/v% penicillin-streptomycin solution added) at $3\times10^4$ cells/600 μL in a 24-well flat-bottom transparent white plate. The obtained nucleic acid-encapsulating particles were added such that the nucleic acid concentration in the medium was 0.03 μg/30 μL. Luciferin was added to the medium at 0.1 mM, and the amount of luciferase protein introduced was evaluated over time using the amount of luminescence. HeLa cells cultured in a medium containing nucleic acid-encapsulating nanoparticles and luciferin were placed in an incubator-type luminometer (Kronos), and the cumulative amount of luminescence was measured every hour. The results are shown in Fig. 1. The vertical axis in Fig. 1 shows luciferase activity (RLU), which is the total cumulative amount of luminescence over 24 hr. In Fig. 1, "3.00.E+07" means "$3.00\times10^7$". Other descriptions similar to "3.00.E+07" also have the same meaning as "3.00.E+07".

As shown in Fig. 1, the nucleic acid-encapsulating nanoparticles of Example 1 prepared using citrate buffer and TBS in combination showed improved nucleic acid introduction efficiency compared to the nucleic acid-encapsulating nanoparticles of Comparative Examples 1 to 6 prepared using other combinations.

Evaluation of storage stability

Measurement of particle size, etc.

The suspensions of nucleic acid-encapsulating nanoparticles obtained in Example 1, Comparative Example 1, and Comparative Example 6 were stored at 4°C for 2 months. The particle size, Polydispersity Index (PdI), and mRNA encapsulation rate of the nucleic acid-encapsulating nanoparticles after storage were analyzed. The particle size and PdI were measured by the dynamic light scattering method using a Zetasizer (registered trademark). The mRNA encapsulation rate was measured by the Ribogreen (registered trademark) assay. The results are shown in Table 3.

[Table 3]

| | Z-Ave (nm) | PdI | Number Mean (nm) | PdI | mRNA encapsulation rate (%) |
|---|---|---|---|---|---|
| Example 1 | 99 | 0.115 | 71 | 0.076 | 79 |
| Comparative Example 1 | 131 | 0.202 | 71 | 0.126 | 72 |
| Comparative Example 6 | 91 | 0.140 | 66 | 0.069 | 80 |

Evaluation of time course changes of nucleic acid introduction efficiency

The suspensions of nucleic acid-encapsulating nanoparticles obtained in Example 1, Comparative Example 1, and Comparative Example 6 were stored at 4°C. On the start date of storage (day 0), the 21st day, the 28th day, and the 57th day of storage, the nucleic acid-encapsulating nanoparticles were applied to HeLa cells to evaluate the nucleic acid introduction efficiency. In detail, HeLa cells were seeded in D-MEM medium (high glucose) (containing L-glutamine and phenol red) (10 v/v % FBS and 1 v/v % penicillin-streptomycin solution added) at $3\times10^4$ cells/600 μL in a 24-well flat-bottom white transparent plate. The obtained nucleic acid-encapsulating particles were added such that the nucleic acid concentration in the medium was 0.03 μg/30 μL. Luciferin was added to the medium at 0.1 mM, and the amount of luciferase protein introduced was evaluated over time using the amount of luminescence. HeLa cells cultured in a medium containing nucleic acid-encapsulating nanoparticles and luciferin were placed in an incubator-type luminometer (Kronos), and the cumulative amount of luminescence was measured every hour. Luciferase activity (RLU), which is the total cumulative luminescence in 24 hr, was calculated and relative RLU (%) was calculated using the following formula:

Relative RLU (%) = 100 × RLU on day x of storage/RLU on day 0 of storage (start of storage)

(in the formula, x is 0, 21, 28, or 57). The results are shown in Fig. 2.

As shown in Fig. 2, the nucleic acid-encapsulating nanoparticles of Example 1 prepared using citrate buffer and TBS in combination suppressed a time-course decrease in the nucleic acid introduction efficiency, compared to the nucleic acid-encapsulating nanoparticles of Comparative Example 1 or 6 prepared using other combinations.

Example 2

As ionic lipid, SS-OP was used. The lipid composition was SS-OP:DOPC:Chol:DMG-PEG2000=52.5:7.5:40:1.5 in molar ratio.

The concentrations of each lipid in the lipid ethanol solution used were as follows.

SS-OP: 1.8 mM
DOPC: 0.3 mM
Chol: 1.3 mM
DMG-PEG2000: 0.05 mM

1200 μL of mRNA-containing buffer solution (mRNA concentration: 5 μg/mL) in which mRNA was dispersed in citrate buffer (total concentration of citric acid and salt thereof: 50 mM, pH:3, 4, 5, or 6) and 450 μL of lipid ethanol solution were weighed into a syringe. Using a NanoAssmblr (registered trademark) ultrafast nanomedicine production device (manufactured by Precision NanoSystems), the mRNA-containing buffer solution and the lipid ethanol solution were mixed under the conditions of flow rate (volume ratio) of mRNA-containing buffer solution:lipid ethanol solution of 3:1, total flow rate: 12 mL/min, inner diameter of micro flow path: 130×300 μm (square), and syringe holder temperature: 25°C to prepare a suspension of nucleic acid-encapsulating nanoparticles (N/P ratio: 68, nucleic acid concentration in suspension: 3.7 μg/mL).

After adding 1000 μL of TBS (total concentration of Tris and salt thereof: 50 mM, NaCl concentration: 150 mM, pH: 7.4) to the recovered suspension, the obtained mixture was transferred to Amicon Ultra 4 and concentrated to about 500 μL by ultrafiltration under centrifugation conditions (25°C, 1000 g, 3 min) .

The obtained concentrate was diluted to 4 mL with the aforementioned TBS and then concentrated again to about 200 μL by ultrafiltration under centrifugation conditions (25°C, 1000 g, 3 min). This dilution and concentration operation was repeated two more times, after which 0.2 mL of the aforementioned TBS was added to the concentrate to obtain a suspension of nucleic acid-encapsulating nanoparticles (nucleic acid concentration in the suspension: 10 μg/mL).

Measurement of particle size, etc.

The particle size, Polydispersity Index (PdI), and mRNA encapsulation rate of the nucleic acid-encapsulating nanoparticles obtained using citrate buffers with different pH values in Example 2 were analyzed. The particle size and PdI were measured by the dynamic light scattering method using a Zetasizer (registered trademark). The mRNA encapsulation rate was measured by the Ribogreen (registered trademark) assay. The results are shown in Table 4.

[Table 4]

| | Z-Ave (nm) | PdI | Number Mean (nm) | PdI | mRNA encapsulation rate (%) |
|---|---|---|---|---|---|
| pH: 3 | 137 | 0.213 | 79 | 0.117 | 71 |
| pH: 4 | 124 | 0.177 | 70 | 0.120 | 78 |
| pH: 5 | 122 | 0.189 | 68 | 0.120 | 78 |
| pH: 6 | 98 | 0.109 | 71 | 0.075 | 60 |

As shown in Table 4, mRNA was encapsulated in LNP irrespective of pH of the citrate buffer and no difference in the encapsulation rate was observed.

Evaluation of nucleic acid introduction efficiency

The nucleic acid-encapsulating nanoparticles obtained in Example 2 and using citrate buffer with different pH were applied to HeLa cells to evaluate the nucleic acid introduction efficiency. In detail, HeLa cells were seeded in D-MEM medium (high glucose) (containing L-glutamine and phenol red) (10 v/v% FBS, 1 v/v% penicillin-streptomycin solution added) at $3\times10^4$ cells/600 μL in a 24-well flat-bottom transparent white plate. The obtained nucleic acid-encapsulating particles were added such that the nucleic acid concentration in the medium was 0.03 μg/60 μL. Luciferin was added to the medium at 0.1 mM, and the amount of luciferase protein introduced was evaluated over time using the amount of luminescence. HeLa cells cultured in a medium containing nucleic acid-encapsulating nanoparticles and luciferin were placed in an incubator-type luminometer (Kronos), and the cumulative amount of luminescence was measured every hour. The results are shown in Fig. 3. The vertical axis in Fig. 3 shows luciferase activity (RLU), which is the total cumulative amount of luminescence over 24 hr. In Fig. 3, "6.0.E+07" means "$6.0\times10^7$". Other descriptions similar to "6.0.E+07" also have the same meaning as "6.0.E+07".

As shown in Fig. 3, the nucleic acid-encapsulating nanoparticles prepared using citrate buffer at pH4 showed improved nucleic acid introduction efficiency compared to the nucleic acid-encapsulating nanoparticles prepared using citrate buffer at other pH.

Example 3

As ionic lipid, SS-OP was used. The lipid composition was SS-OP:DOPC:Chol:DMG-PEG2000=52.5:7.5:40:1.5 in molar ratio.

The concentrations of each lipid in the lipid ethanol solution used were as follows.

SS-OP: 3.5 mM
DOPC: 0.5 mM
Chol: 2.7 mM
DMG-PEG2000: 0.1 mM

1200 μL of mRNA-containing buffer solution (mRNA concentration: (1) 147 μg/mL, (2) 122 μg/mL, (3) 50 μg/mL, (4) 12.5 μg/mL, (5) 6.3 μg/mL, (6) 3.1 μg/mL) in which mRNA was dispersed in citrate buffer (total concentration of citric

acid and salt thereof: 20 mM, pH: 5.0) and 450 μL of lipid ethanol solution were weighed into a syringe. Using a NanoAssmblr (registered trademark) ultrafast nanomedicine production device (manufactured by Precision NanoSystems) and under the conditions of flow rate of mRNA-containing buffer solution:lipid ethanol solution of 3:1, total flow rate: 4 mL/min, inner diameter of micro flow path: 130×300 μm (square), and syringe holder temperature: 25°C, a suspension of nucleic acid-encapsulating nanoparticles (N/P ratio: (1) 5.7, (2) 7, (3) 17, (4) 68, (5) 136, (6) 272, nucleic acid concentration in suspension: (1) 20.8 μg/mL, (2) 19.2 μg/mL, (3) 7.7 μg/mL, (4) 3.0 μg/mL, (5) 1.6 μg/mL, (6) 0.9 μg/mL) was prepared.

After adding 1000 μL of TBS (total concentration of Tris and salt thereof: 50 mM, NaCl concentration: 150 mM, pH: 7.4) to the recovered suspension, the obtained mixture was transferred to Amicon Ultra 4 and concentrated to about 500 μL by ultrafiltration under centrifugation conditions (25°C, 1000 g, 3 min).

The obtained concentrate was diluted to 4 mL with the aforementioned TBS and then concentrated again to about 150 to 1000 μL by ultrafiltration under centrifugation conditions (25°C, 1000 g, 3 min). This dilution and concentration operation was repeated two more times, after which 0.1 to 3.5 mL of the aforementioned TBS was added to the concentrate to obtain a suspension of nucleic acid-encapsulating nanoparticles (nucleic acid concentration in the suspension: (1) - (6) 10 μg/mL).

Measurement of particle size, etc.

The particle size, Polydispersity Index (PdI), and mRNA encapsulation rate of the nucleic acid-encapsulating nanoparticles obtained in Example 3 under conditions with different N/P ratio were analyzed. The particle size and PdI were measured by the dynamic light scattering method using a Zetasizer (registered trademark). The mRNA encapsulation rate was measured by the Ribogreen (registered trademark) assay. The results are shown in Table 5.

[Table 5]

| N/P ratio | Z-Ave (nm) | PdI | Number Mean (nm) | PdI | mRNA encapsulation rate (%) |
|---|---|---|---|---|---|
| 5.7 | 126 | 0.142 | 83 | 0.107 | 41 |
| 7 | 120 | 0.124 | 82 | 0.097 | 48 |
| 17 | 108 | 0.115 | 69 | 0.098 | 77 |
| 68 | 107 | 0.099 | 73 | 0.089 | 83 |
| 136 | 105 | 0.108 | 77 | 0.076 | 82 |
| 272 | 106 | 0.127 | 72 | 0.090 | 82 |

Evaluation of nucleic acid introduction efficiency

The nucleic acid-encapsulating nanoparticles obtained in Example 3 under conditions with different N/P ratio were applied to HeLa cells to evaluate the nucleic acid introduction efficiency. In detail, HeLa cells were seeded in D-MEM medium (high glucose) (containing L-glutamine and phenol red) (10 v/v% FBS, 1 v/v% penicillin-streptomycin solution added) at $3\times10^4$ cells/600 μL in a 24-well flat-bottom transparent white plate. The obtained nucleic acid-encapsulating particles were added such that the nucleic acid concentration in the medium was 0.03 μg/60 μL. Luciferin was added to the medium at 0.1 mM, and the amount of luciferase protein introduced was evaluated over time using the amount of luminescence. HeLa cells cultured in a medium containing nucleic acid-encapsulating nanoparticles and luciferin were placed in an incubator-type luminometer (Kronos), and the cumulative amount of luminescence was measured every hour. The results are shown in Fig. 4. The vertical axis in Fig. 4 shows luciferase activity (RLU), which is the total cumulative amount of luminescence over 24 hr.

Example 4

As ionic lipid, SS-EC was used. The lipid composition was SS-EC:DOPE:Chol:DMG-PEG2000=60:30:10:3 in molar ratio.

The concentrations of each lipid in the lipid ethanol solution used were as follows.

SS-EC: 4.0 mM
DOPE: 2.0 mM
Chol: 0.7 mM
DMG-PEG2000: 0.2 mM

1300 μL of mRNA-containing buffer solution (mRNA concentration: 5 μg/mL) in which mRNA was dispersed in citrate buffer (total concentration of citric acid and salt thereof: 20 mM, pH: 5.0) and 600 μL of lipid ethanol solution were weighed into a syringe. Using a NanoAssmblr (registered trademark) ultrafast nanomedicine production device (manufactured by Precision NanoSystems), and under the conditions of flow rate of mRNA-containing buffer solution:lipid ethanol solution of 3:1, total flow rate: 12 mL/min, inner diameter of micro flow path: 130×300 μm (square), and syringe holder temperature: 25°C, a suspension of nucleic acid-encapsulating nanoparticles (N/P ratio: 45, nucleic acid concentration in suspension: 15 μg/mL) was prepared.

After adding 1000 μL of TBS (total concentration of Tris and salt thereof: 50 mM, NaCl concentration: 150 mM, pH: 7.4) to the recovered suspension, the obtained mixture was transferred to Amicon Ultra 4 and concentrated to about 1000 μL by ultrafiltration under centrifugation conditions (25°C, 1000 g, 3 min).

The obtained concentrate was diluted to 4 mL with the aforementioned TBS and then concentrated again to about 750 μL by ultrafiltration under centrifugation conditions (25°C, 1000 g, 3 min). This dilution and concentration operation was repeated two more times, after which 0.75 mL of the aforementioned TBS was added to the concentrate to obtain a suspension of nucleic acid-encapsulating nanoparticles (nucleic acid concentration in the suspension: 10 μg/mL).

Comparative Example 7

As ionic lipid, SS-EC was used. The lipid composition was SS-EC:DOPE:Chol:DMG-PEG2000=60:30:10:3 in molar ratio.

The concentrations of each lipid in the lipid ethanol solution used were as follows.

SS-EC: 4.0 mM
DOPE: 2.0 mM
Chol: 0.7 mM
DMG-PEG2000: 0.2 mM

1300 μL of mRNA-containing buffer solution (mRNA concentration: 5 μg/mL) in which mRNA was dispersed in malic acid buffer (pH: 3.0) and 600 μL of lipid ethanol solution were weighed into a syringe. Using a NanoAssmblr (registered trademark) ultrafast nanomedicine production device (manufactured by Precision NanoSystems), the mRNA-containing buffer solution and the lipid ethanol solution were mixed under the conditions of flow rate of mRNA-containing buffer solution:lipid ethanol solution of 3:1, total flow rate: 12 mL/min, inner diameter of micro flow path: 130×300 μm (square), and syringe holder temperature: 25°C to prepare a suspension of nucleic acid-encapsulating nanoparticles (N/P ratio: 45, nucleic acid concentration in suspension: 15 μg/mL).

After adding 1000 μL of MES buffer (pH 6.5) to the recovered suspension, the obtained mixture was transferred to Amicon Ultra 4 and concentrated to about 1000 μL by ultrafiltration under centrifugation conditions (25°C, 1000 g, 3 min).

The obtained concentrate was diluted to 4 mL with PBS (pH:7.4) and then concentrated again to about 750 μL by ultrafiltration under centrifugation conditions (25°C, 1000 g, 3 min). This dilution and concentration operation was repeated two more times, after which 0.75 mL of the aforementioned PBS was added to the concentrate to obtain a suspension of nucleic acid-encapsulating nanoparticles (nucleic acid concentration in the suspension: 10 μg/mL).

Measurement of particle size, etc.

The particle size, Polydispersity Index (PdI), and mRNA encapsulation rate of the nucleic acid-encapsulating nanoparticles obtained in Example 4 and Comparative Example 7 were analyzed. The particle size and PdI were measured by the dynamic light scattering method using a Zetasizer (registered trademark). The mRNA encapsulation rate was measured by the Ribogreen (registered trademark) assay. The results are shown in Table 6.

[Table 6]

| | Z-Ave (nm) | PdI | Number Mean (nm) | PdI | mRNA encapsulation rate (%) |
|---|---|---|---|---|---|
| Example 4 | 73 | 0.106 | 50 | 0.072 | 72 |
| Comparative Example 7 | 65 | 0.102 | 44 | 0.072 | 75 |

As shown in Table 6, mRNA was encapsulated in LNP in Example 4, in which SS-EC was used as the ionic lipid, and no difference in the mRNA encapsulation rate was observed between Example 4 and Comparative Example 7, which used other conditions.

Evaluation of time course changes of nucleic acid introduction efficiency

The suspensions of nucleic acid-encapsulating nanoparticles obtained in Example 4 and Comparative Example 7 were stored at 4°C. On the start date of storage (day 0), the 7th day, and the 14th day of storage, the nucleic acid-encapsulating nanoparticles were applied to HeLa cells to evaluate the nucleic acid introduction efficiency. In detail, HeLa cells were seeded in D-MEM medium (high glucose) (containing L-glutamine and phenol red) (10 v/v % FBS and 1 v/v % penicillin-streptomycin solution added) at $3\times10^4$ cells/600 μL in a 24-well flat-bottom white transparent plate. The obtained nucleic acid-encapsulating particles were added such that the nucleic acid concentration in the medium was 0.03 μg/30 μL. Luciferin was added to the medium at 0.1 mM, and the amount of luciferase protein introduced was evaluated over time using the amount of luminescence. HeLa cells cultured in a medium containing nucleic acid-encapsulating nanoparticles and luciferin were placed in an incubator-type luminometer (Kronos), and the cumulative amount of luminescence was measured every hour. The results are shown in Fig. 5. The vertical axis in Fig. 5 shows luciferase activity (RLU), which is the total cumulative amount of luminescence over 24 hr. The three bar graphs in each of Example 4 and Comparative Example 7 in Fig. 5 show, from the left, the results on the start date of storage (day 0), the seventh day of storage, and the 14th day of storage. In Fig. 5, "8.00.E+06" means "$8.00\times10^6$". Other descriptions similar to "8.00.E+06" also have the same meaning as "8.00.E+06".

As shown in Fig. 5, the nucleic acid-encapsulating nanoparticles of Example 4 prepared using citrate buffer and TBS in combination improved the nucleic acid introduction efficiency and suppressed a time-course decrease in the nucleic acid introduction efficiency, compared to the nucleic acid-encapsulating nanoparticles of Comparative Example 7 prepared using other combinations.

Example 5

As ionic lipid, SS-OP and SS-EC were used. The lipid composition was SS-OP:SS-EC:DOPE:Chol:DMG-PEG2000=32.5:20:7.5:40:1.5 in molar ratio.

The concentrations of each lipid in the lipid ethanol solution used were as follows.

SS-OP: 2.2 mM
SS-EC: 1.3 mM
DOPE: 0.5 mM
Chol: 2.7 mM
DMG-PEG2000: 0.1 mM

1300 μL of mRNA-containing buffer solution (mRNA concentration: 5 μg/mL) in which mRNA was dispersed in citrate buffer (total concentration of citric acid and salt thereof: 20 mM, pH: 5.0) and 600 μL of lipid ethanol solution were weighed into a syringe. Using a NanoAssmblr (registered trademark) ultrafast nanomedicine production device (manufactured by Precision NanoSystems), the mRNA-containing buffer solution and the lipid ethanol solution were mixed under the conditions of flow rate of mRNA-containing buffer solution:lipid ethanol solution of 3:1, total flow rate: 12 mL/min, inner diameter of micro flow path: 130×300 μm (square), and syringe holder temperature: 25°C to prepare a suspension of nucleic acid-encapsulating nanoparticles (N/P ratio: 34, nucleic acid concentration in suspension: 20 μg/mL).

After adding 1000 μL of TBS (total concentration of Tris and salt thereof: 50 mM, NaCl concentration: 150 mM, pH: 7.4) to the recovered suspension, the obtained mixture was transferred to Amicon Ultra 4 and concentrated to about 1000 μL by ultrafiltration under centrifugation conditions (25°C, 1000 g, 3 min).

The obtained concentrate was diluted to 4 mL with the aforementioned TBS and then concentrated again to about 750 μL by ultrafiltration under centrifugation conditions (25°C, 1000 g, 3 min). This dilution and concentration operation was repeated two more times, after which 0.75 mL of the aforementioned TBS was added to the concentrate to obtain a suspension of nucleic acid-encapsulating nanoparticles (nucleic acid concentration in the suspension: 10 μg/mL).

Comparative Example 8

As ionic lipid, SS-OP and SS-EC were used. The lipid composition was SS-OP:SS-EC:DOPE:Chol:DMG-PEG2000=32.5:20:7.5:40:1.5 in molar ratio.

The concentrations of each lipid in the lipid ethanol solution used were as follows.

SS-OP: 2.2 mM
SS-EC: 1.3 mM
DOPE: 0.5 mM
Chol: 2.7 mM

DMG-PEG2000: 0.1 mM

1300 μL of mRNA-containing buffer solution (mRNA concentration: 5 μg/mL) in which mRNA was dispersed in malic acid buffer (total concentration of malic acid and salt thereof: 20 mM, pH: 3.0) and 600 μL of lipid ethanol solution were weighed into a syringe. Using a NanoAssmblr (registered trademark) ultrafast nanomedicine production device (manufactured by Precision NanoSystems), the mRNA-containing buffer solution and the lipid ethanol solution were mixed under the conditions of flow rate of mRNA-containing buffer solution:lipid ethanol solution of 3:1, total flow rate: 12 mL/min, inner diameter of micro flow path: 130×300 μm (square), and syringe holder temperature: 25°C to prepare a suspension of nucleic acid-encapsulating nanoparticles (N/P ratio: 34, nucleic acid concentration in suspension: 20 μg/mL).
After adding 1000 μL of MES buffer (pH 6.5) to the recovered suspension, the obtained mixture was transferred to Amicon Ultra 4 and concentrated to about 1000 μL by ultrafiltration under centrifugation conditions (25°C, 1000 g, 3 min).
The obtained concentrate was diluted to 4 mL with PBS (pH:7.4) and then concentrated again to about 750 μL by ultrafiltration under centrifugation conditions (25°C, 1000 g, 3 min). This dilution and concentration operation was repeated two more times, after which 0.75 mL of the aforementioned PBS was added to the concentrate to obtain a suspension of nucleic acid-encapsulating nanoparticles (nucleic acid concentration in the suspension: 10 μg/mL).

Measurement of particle size, etc.

The particle size, Polydispersity Index (PdI), and mRNA encapsulation rate of the nucleic acid-encapsulating nanoparticles obtained in Example 5 and Comparative Example 8 were analyzed. The particle size and PdI were measured by the dynamic light scattering method using a Zetasizer (registered trademark). The mRNA encapsulation rate was measured by the Ribogreen (registered trademark) assay. The results are shown in Table 7.

[Table 7]

| | Z-Ave (nm) | PdI | Number Mean (nm) | PdI | mRNA encapsulation rate (%) |
|---|---|---|---|---|---|
| Example 5 | 93 | 0.112 | 62 | 0.086 | 86 |
| Comparative Example 8 | 96 | 0.136 | 62 | 0.087 | 86 |

As shown in Table 7, mRNA was encapsulated in LNP in Example 5, in which SS-OP and SS-EC were used as the ionic lipid, and no difference in the mRNA encapsulation rate was observed between Example 5 and Comparative Example 8, which used other conditions.

Evaluation of nucleic acid introduction efficiency

The nucleic acid-encapsulating nanoparticles obtained in Example 5 and Comparative Example 8 were applied to HeLa cells to evaluate the nucleic acid introduction efficiency. In detail, HeLa cells were seeded in D-MEM medium (high glucose) (containing L-glutamine and phenol red) (10 v/v% FBS, 1 v/v% penicillin-streptomycin solution added) at $3\times10^4$ cells/600 μL in a 24-well flat-bottom transparent white plate. The obtained nucleic acid-encapsulating particles were added such that the nucleic acid concentration in the medium was 0.4 μg/200 μL. Luciferin was added to the medium at 0.1 mM, and the amount of luciferase protein introduced was evaluated over time using the amount of luminescence. HeLa cells cultured in a medium containing nucleic acid-encapsulating nanoparticles and luciferin were placed in an incubator-type luminometer (Kronos), and the cumulative amount of luminescence was measured every hour. The results are shown in Fig. 6. The vertical axis in Fig. 6 shows luciferase activity (RLU), which is the total cumulative amount of luminescence over 24 hr. In Fig. 6, "4.40.E+07" means "$4.40\times10^7$". Other descriptions similar to "4.40.E+07" also have the same meaning as "4.40.E+07".
As shown in Fig. 6, the nucleic acid-encapsulating nanoparticles of Example 5, which used a combination of citrate buffer and TBS, showed improved nucleic acid introduction efficiency compared to the nucleic acid-encapsulating nanoparticles of Comparative Example 8, which used other combination.

Evaluation of time course changes in nucleic acid introduction efficiency in vivo

The nucleic acid-encapsulating nanoparticles obtained in Example 1, Comparative Example 1, and Comparative Example 6 were stored at 4°C. On day 1 and day 28 of storage, 200 μL was administered via the tail vein of 6-week-old female C57BL/6J mice (mRNA dose: 0.1 μg per mouse). Four hours after administration, the liver was removed and the expression level of luciferase protein was evaluated using the IVIS Imaging System. The results are shown in Fig. 7. The

vertical axis in Fig. 7 shows luciferase activity (RLU), which is the total cumulative amount of luminescence over 24 hr. In Fig. 7, "1.4E+10" means "$1.4\times10^{10}$". Other descriptions similar to "1.4E+10" also have the same meaning as "1.4E+10".

As shown in Fig. 7, only in the nucleic acid-encapsulating nanoparticles of Example 1, in which citrate buffer and TBS were used in combination, among the nucleic acid-encapsulating nanoparticles of Example 1, Comparative Example 1, and Comparative Example 6, the nucleic acid introduction efficiency on day 28 of storage was equivalent to that on day 1 of storage.

Example 6

As ionic lipid, compound (5) was used. The lipid composition was compound (5):DOPC:Chol:DMG-PEG2000=49:7.5:42.5:1 in molar ratio.

The concentrations of each lipid in the lipid ethanol solution used were as follows.

compound (5): 5.0 mM
DOPC: 5.0 mM
Chol: 10 mM
DMG-PEG2000: 0.5 mM

1300 μL of mRNA-containing buffer solution (mRNA concentration: 5 μg/mL) in which mRNA was dispersed in citrate buffer (total concentration of citric acid and salt thereof: 20 mM, pH: 5.0) and 600 μL of lipid ethanol solution were weighed into a syringe. Using a NanoAssmblr (registered trademark) ultrafast nanomedicine production device (manufactured by Precision NanoSystems), and under the conditions of flow rate of mRNA-containing buffer solution:lipid ethanol solution of 3:1, total flow rate: 12 mL/min, inner diameter of micro flow path: 130×300 μm (square), and syringe holder temperature: 25°C, a suspension of nucleic acid-encapsulating nanoparticles (N/P ratio: 45, nucleic acid concentration in suspension: 3.7 μg/mL) was prepared.

After adding 1000 μL of TBS (total concentration of Tris and salt thereof: 50 mM, NaCl concentration: 150 mM, pH: 7.6) to the recovered suspension, the obtained mixture was transferred to Amicon Ultra 4 and concentrated to about 1000 μL by ultrafiltration under centrifugation conditions (25°C, 1000 g, 3 min).

The obtained concentrate was diluted to 4 mL with the aforementioned TBS and then concentrated again to about 200 μL by ultrafiltration under centrifugation conditions (25°C, 1000 g, 3 min). This dilution and concentration operation was repeated two more times, after which 0.20 mL of the aforementioned TBS was added to the concentrate to obtain a suspension of nucleic acid-encapsulating nanoparticles (nucleic acid concentration in the suspension: 10 μg/mL).

Comparative Example 9

As ionic lipid, compound (5) was used. The lipid composition was compound (5) :DOPC:Chol:DMG-PEG2000=49:7.5:42.5:1 in molar ratio.

The concentrations of each lipid in the lipid ethanol solution used were as follows.

compound (5): 5.0 mM
DOPC: 5.0 mM
Chol: 10 mM
DMG-PEG2000: 0.5 mM

1300 μL of mRNA-containing buffer solution (mRNA concentration: 5 μg/mL) in which mRNA was dispersed in malic acid buffer (total concentration of malic acid and salt thereof: 20 mM, pH: 3.0) and 600 μL of lipid ethanol solution were weighed into a syringe. Using a NanoAssmblr (registered trademark) ultrafast nanomedicine production device (manufactured by Precision NanoSystems), the mRNA-containing buffer solution and the lipid ethanol solution were mixed under the conditions of flow rate of mRNA-containing buffer solution:lipid ethanol solution of 3:1, total flow rate: 12 mL/min, inner diameter of micro flow path: 130×300 μm (square), and syringe holder temperature: 25°C to prepare nucleic acid-encapsulating nanoparticles (N/P ratio: 34, nucleic acid concentration in suspension: 3.7 μg/mL).

After adding 1000 μL of MES buffer (pH 6.5) to the recovered suspension, the obtained mixture was transferred to Amicon Ultra 4 and concentrated to about 1000 μL by ultrafiltration under centrifugation conditions (25°C, 1000 g, 3 min).

The obtained concentrate was diluted to 4 mL with PBS (pH:7.4) and then concentrated again to about 750 μL by ultrafiltration under centrifugation conditions (25°C, 1000 g, 3 min). This dilution and concentration operation was repeated two more times, after which 0.75 mL of the aforementioned PBS was added to the concentrate to obtain a suspension of nucleic acid-encapsulating nanoparticles (nucleic acid concentration in the suspension: 10 μg/mL).

Measurement of particle size, etc.

The particle size, Polydispersity Index (PdI), and mRNA encapsulation rate of the nucleic acid-encapsulating nanoparticles obtained in Example 6 and Comparative Example 9 were analyzed. The particle size and PdI were measured by the dynamic light scattering method using a Zetasizer (registered trademark). The mRNA encapsulation rate was measured by the Ribogreen (registered trademark) assay. The results are shown in Table 8.

[Table 8]

| | Z-Ave (nm) | PdI | Number Mean (nm) | PdI | mRNA encapsulation rate (%) |
|---|---|---|---|---|---|
| Example 6 | 127 | 0.09 | 95 | 0.09 | 80 |
| Comparative Example 9 | 89 | 0.09 | 66 | 0.07 | 85 |

As shown in Table 8, mRNA was encapsulated in LNP in Example 6, in which compound (5) was used as the ionic lipid, and no difference in the mRNA encapsulation rate was observed between Example 6 and Comparative Example 9, which used other conditions.

Evaluation of nucleic acid introduction efficiency

The nucleic acid-encapsulating nanoparticles obtained in Example 6 and Comparative Example 9 were applied to HeLa cells to evaluate the nucleic acid introduction efficiency. In detail, HeLa cells were seeded in D-MEM medium (high glucose) (containing L-glutamine and phenol red) (10 v/v% FBS, 1 v/v% penicillin-streptomycin solution added) at $3\times10^4$ cells/600 μL in a 24-well flat-bottom transparent white plate. The obtained nucleic acid-encapsulating particles were added such that the nucleic acid concentration in the medium was 0.4 μg/200 μL. Luciferin was added to the medium at 0.1 mM, and the amount of luciferase protein introduced was evaluated over time using the amount of luminescence. HeLa cells cultured in a medium containing nucleic acid-encapsulating nanoparticles and luciferin were placed in an incubator-type luminometer (Kronos), and the cumulative amount of luminescence was measured every hour. The results are shown in Fig. 8. The vertical axis in Fig. 8 shows luciferase activity (RLU), which is the total cumulative amount of luminescence over 24 hr. In Fig. 8, "1.40E+08" means "$1.40\times10^8$". Other descriptions similar to "1.40E+08" also have the same meaning as "1.40E+08".

As shown in Fig. 8, the nucleic acid-encapsulating nanoparticles of Example 6, which used a combination of citrate buffer and TBS, showed improved nucleic acid introduction efficiency compared to the nucleic acid-encapsulating nanoparticles of Comparative Example 9, which used other combination.

[Industrial Applicability]

According to the method of the present invention, a suspension of nucleic acid-encapsulating lipid nanoparticles, which is superior in storage stability, can be produced. The nucleic acid-encapsulating lipid nanoparticles obtained by the method of the present invention are useful for nucleic acid medicines, gene therapy, and biochemical experiments.

This application is based on a patent application No. 2023-053865 filed in Japan, the contents of which are encompassed in full herein.

## Claims

**1.** A method for producing nucleic acid-encapsulating lipid nanoparticles, comprising the following steps (a) and (b):

step (a) of mixing an alcohol solution containing an ionic lipid having a tertiary amino group, a sterol, and a PEG lipid with a citrate buffer having pH 3 to 6.5 in which nucleic acid is dispersed to prepare a suspension of nucleic acid-encapsulating lipid nanoparticles; and

step (b) of exchanging a dispersion medium of the suspension for a Tris buffer having pH 5.2 to 9.0 by concentrating the suspension of nucleic acid-encapsulating lipid nanoparticles by ultrafiltration and diluting same with the Tris buffer.

**2.** The method according to claim 1, wherein a total concentration of citric acid and a salt thereof in the citrate buffer used in step (a) is 10 to 100 mM, and a total concentration of trishydroxymethylaminomethane and a salt thereof in the Tris buffer used in step (b) is 10 to 200 mM.

3. The method according to claim 1, wherein the alcohol solution used in step (a) further comprises a phospholipid.

4. The method according to claim 1, wherein the ionic lipid is a compound represented by the formula (1):

[Chem. 1]

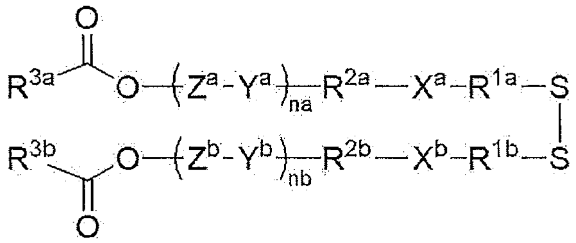

(1)

(in the formula (1),

$R^{1a}$ and $R^{1b}$ are each independently an alkylene group having 1 to 6 carbon atoms,
$X^a$ and $X^b$ are each independently an acyclic alkyl tertiary amino group having 1 to 6 carbon atoms and 1 tertiary amino group, or a cyclic alkylene tertiary amino group having 2 to 5 carbon atoms and 1 or 2 tertiary amino groups,
$R^{2a}$ and $R^{2b}$ are each independently an alkylene group having 1 to 8 carbon atoms or an oxydialkylene group having 2 to 8 carbon atoms,
$Y^a$ and $Y^b$ are each independently an ester bond, an amide bond, a carbamate bond, an ether bond, or a urea bond,
$Z^a$ and $Z^b$ are each independently a divalent group derived from an aromatic compound having 3 to 16 carbon atoms and at least one aromatic ring, and optionally having a hetero atom,
na and nb are each independently 0 or 1,
$R^{3a}$ and $R^{3b}$ are each independently a residue derived from a reaction product of a liposoluble vitamin having a hydroxyl group, and succinic anhydride or glutaric anhydride, a residue derived from a reaction product of a sterol derivative having a hydroxyl group, and succinic anhydride or glutaric anhydride, an aliphatic hydrocarbon group having 1 to 40 carbon atoms, an alkyl group having a cyclopropane ring and having 3 to 40 carbon atoms, or a group represented by the formula (3c):

$$R^{3c}\text{-O-CO-}(CH_2)_a\text{-}* \qquad (3c)$$

(in the formula (3c),
* is a bonding position,
$R^{3c}$ is an aliphatic hydrocarbon group having 2 to 20 carbon atoms, and
a is an integer of 2 to 10)).

5. The method according to claim 1, wherein a molar ratio of the total amino groups of the ionic lipid to the phosphate groups of the nucleic acid (total amino groups of ionic lipid/phosphate groups of nucleic acid) used in step (a) is not less than 7.

6. A method for introducing a nucleic acid into a cell, comprising contacting a nucleic acid-encapsulating lipid nanoparticle produced by the method according to any one of claims 1 to 5 with the cell in vitro.

7. A method for introducing a nucleic acid into a target cell of a living organism, comprising administering nucleic acid-encapsulating lipid nanoparticles produced by the method according to any one of claims 1 to 5 to the living organism.

8. A method for producing a pharmaceutical composition, comprising the method according to any one of claims 1 to 5.

[Fig. 1]

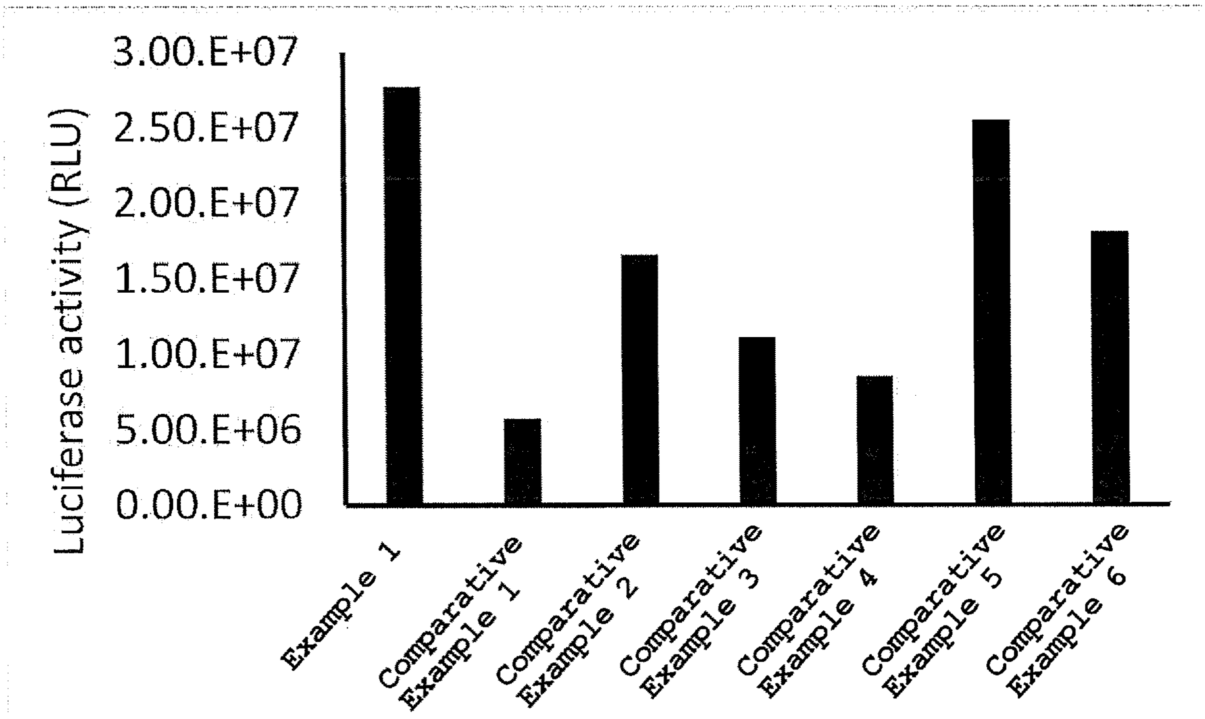
Luciferase activity (RLU)
3.00.E+07
2.50.E+07
2.00.E+07
1.50.E+07
1.00.E+07
5.00.E+06
0.00.E+00
Example 1
Comparative Example 1
Comparative Example 2
Comparative Example 3
Comparative Example 4
Comparative Example 5
Comparative Example 6

[Fig. 2]

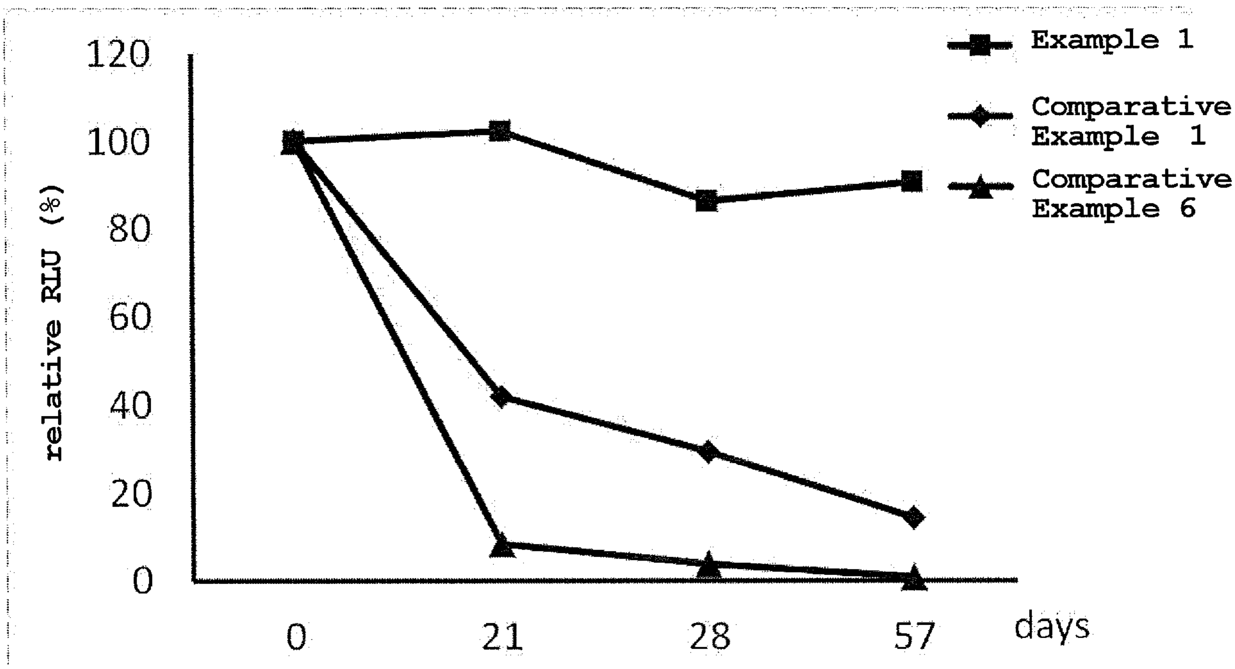
relative RLU (%)
120
100
80
60
40
20
0
0
21
28
57
days
Example 1
Comparative Example 1
Comparative Example 6

[Fig. 3]

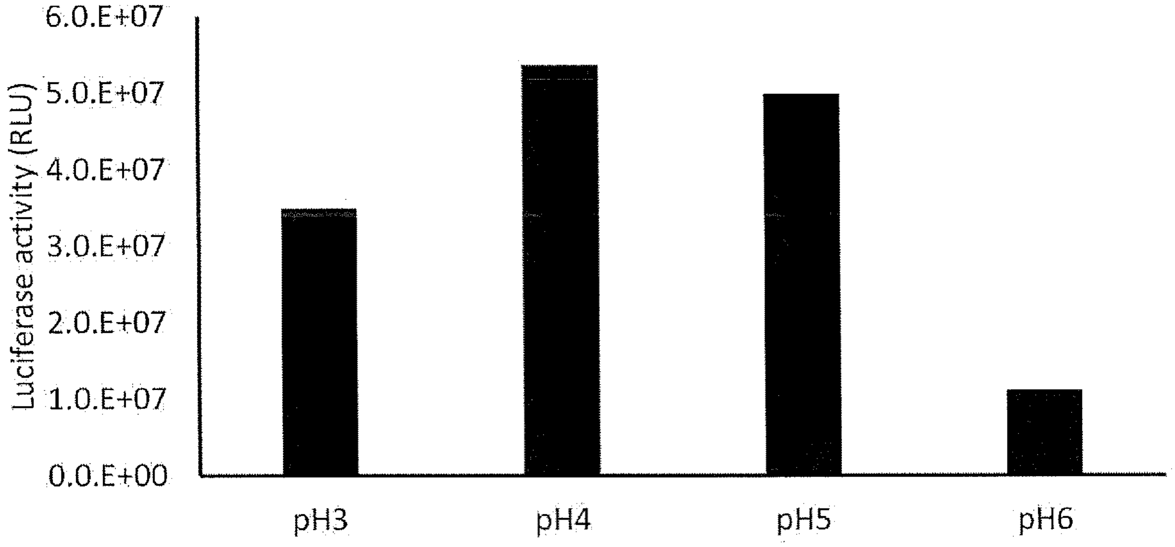
6.0.E+07
5.0.E+07
4.0.E+07
3.0.E+07
2.0.E+07
1.0.E+07
0.0.E+00
Luciferase activity (RLU)
pH3
pH4
pH5
pH6

[Fig. 4]

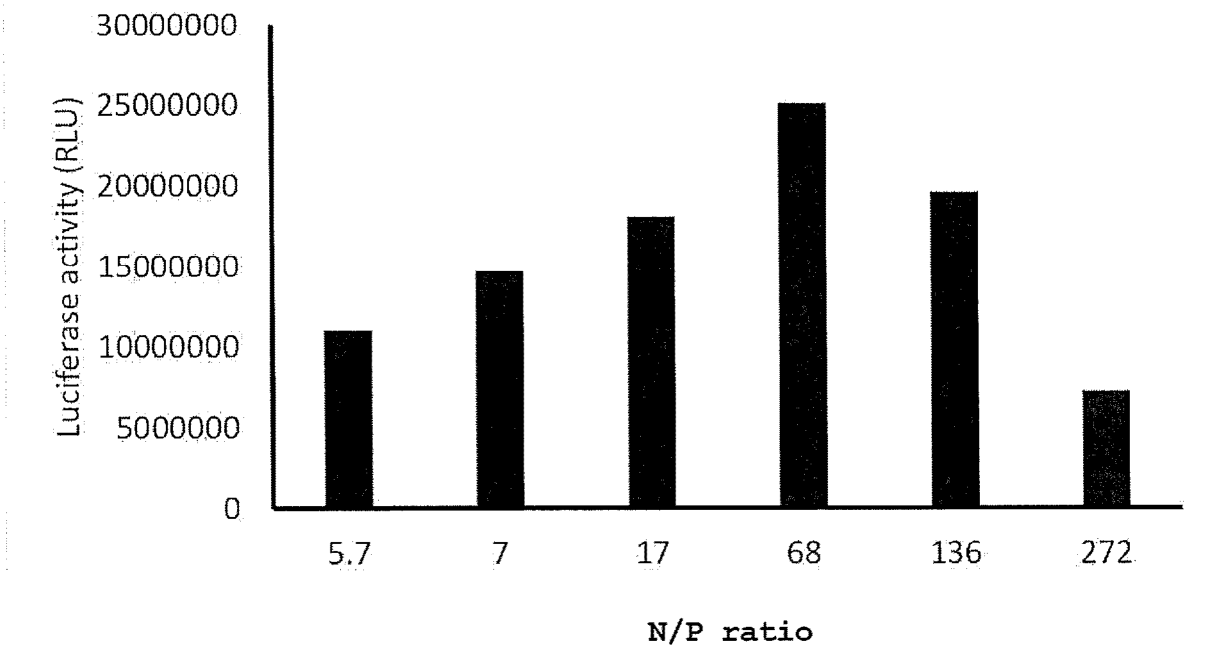
30000000
25000000
20000000
15000000
10000000
5000000
0
Luciferase activity (RLU)
5.7
7
17
68
136
272
N/P ratio

[Fig. 5]

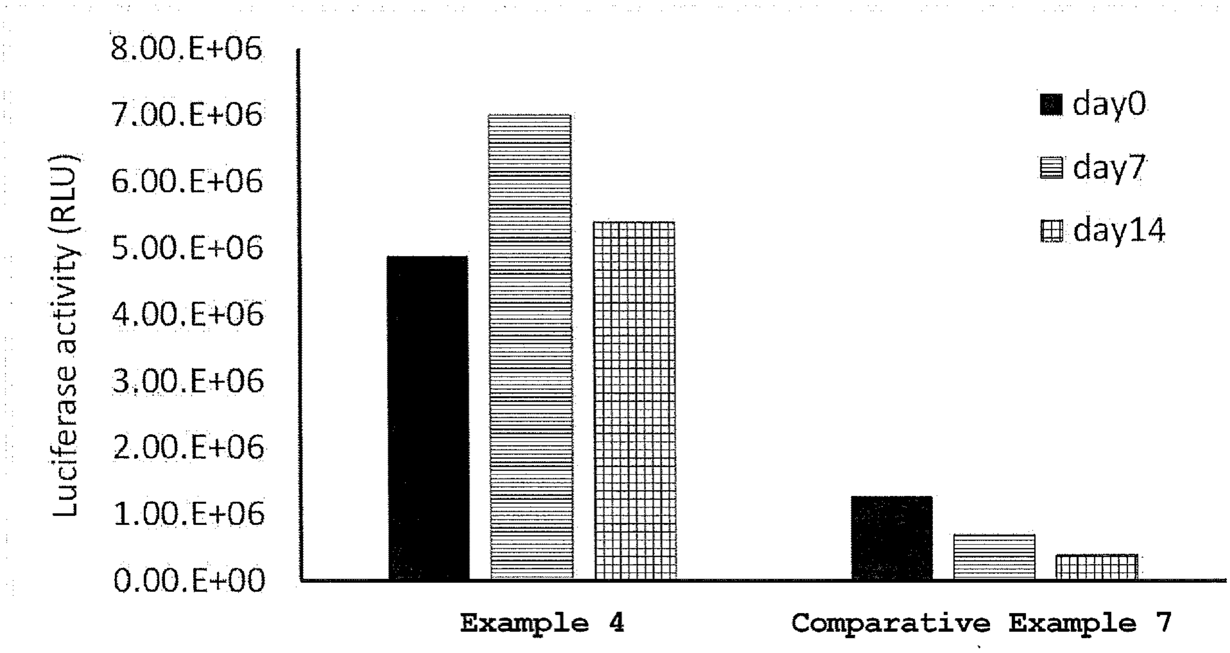
8.00.E+06
7.00.E+06
6.00.E+06
5.00.E+06
4.00.E+06
3.00.E+06
2.00.E+06
1.00.E+06
0.00.E+00
Luciferase activity (RLU)
day0
day7
day14
Example 4
Comparative Example 7

[Fig. 6]

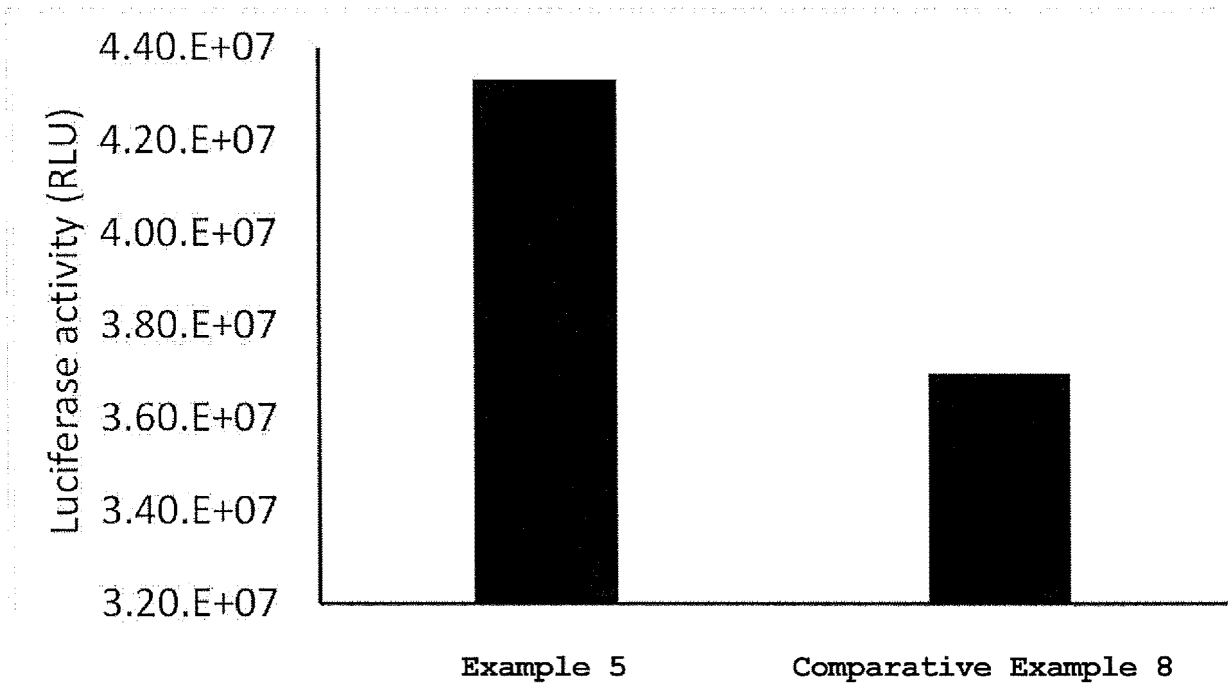
4.40.E+07
4.20.E+07
4.00.E+07
3.80.E+07
3.60.E+07
3.40.E+07
3.20.E+07
Luciferase activity (RLU)
Example 5
Comparative Example 8

[Fig. 7]

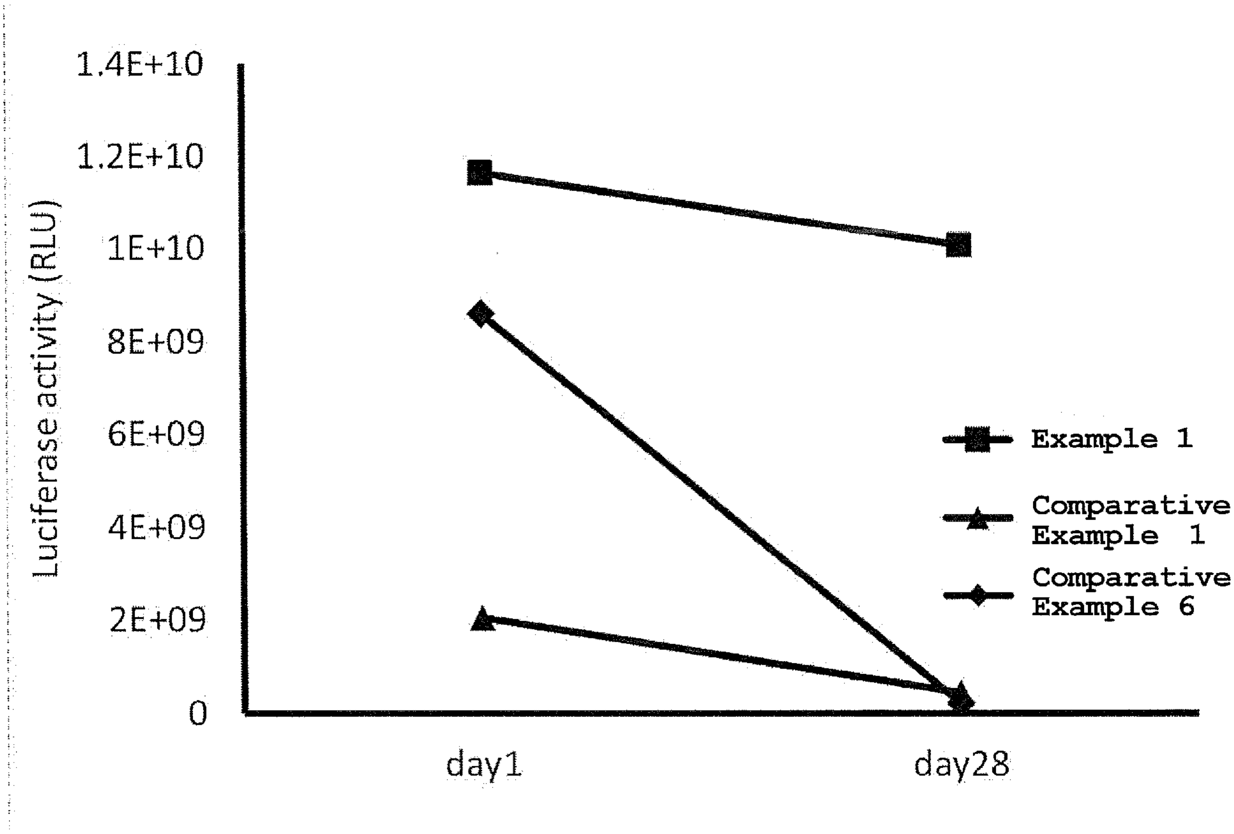
1.4E+10
1.2E+10
1E+10
8E+09
6E+09
4E+09
2E+09
0
Luciferase activity (RLU)
day1
day28
Example 1
Comparative Example 1
Comparative Example 6

[Fig. 8]

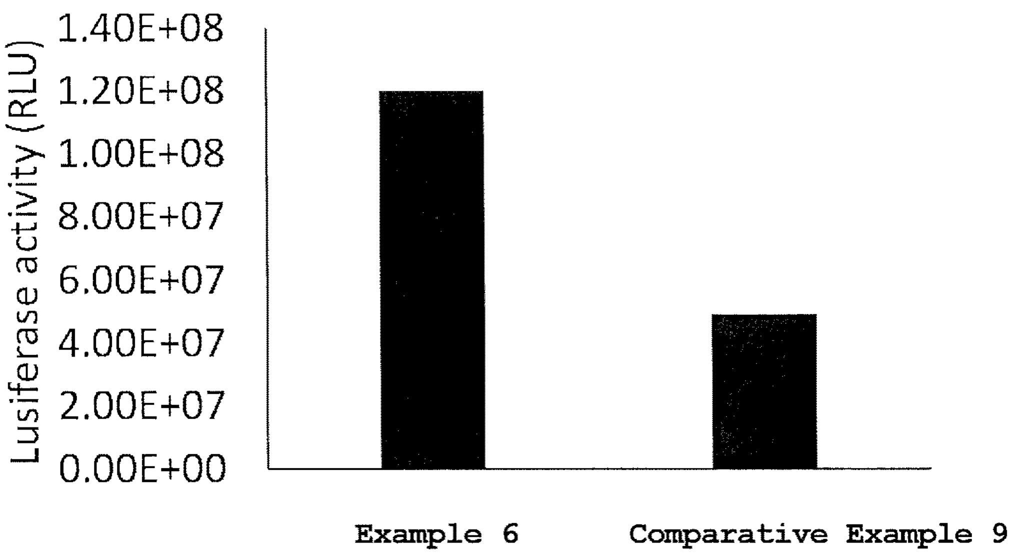
1.40E+08
1.20E+08
1.00E+08
8.00E+07
6.00E+07
4.00E+07
2.00E+07
0.00E+00
Lusiferase activity (RLU)
Example 6
Comparative Example 9

# INTERNATIONAL SEARCH REPORT

International application No.
**PCT/JP2024/010857**

## A. CLASSIFICATION OF SUBJECT MATTER

***A61K 31/7088***(2006.01)i; ***A61K 9/51***(2006.01)i; ***A61K 47/12***(2006.01)i; ***A61K 47/18***(2017.01)i; ***A61K 47/24***(2006.01)i; ***A61K 47/28***(2006.01)i; ***A61K 48/00***(2006.01)i
FI: A61K31/7088; A61K48/00; A61K47/28; A61K47/18; A61K47/12; A61K47/24; A61K9/51

According to International Patent Classification (IPC) or to both national classification and IPC

## B. FIELDS SEARCHED

Minimum documentation searched (classification system followed by classification symbols)

A61K31/7088; A61K9/51; A61K47/12; A61K47/18; A61K47/24; A61K47/28; A61K48/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

## C. DOCUMENTS CONSIDERED TO BE RELEVANT

| Category* | Citation of document, with indication, where appropriate, of the relevant passages | Relevant to claim No. |
|---|---|---|
| Y | WO 2022/101470 A1 (BIONTECH SE) 19 May 2022 (2022-05-19) claims, examples | 1-8 |
| Y | WO 2022/060871 A1 (VERVE THERAPEUTICS, INC.) 24 March 2022 (2022-03-24) claims, paragraphs [0252]-[0256], examples | 1-8 |
| Y | JP 2020-521781 A (GLAXOSMITHKLINE BIOLOGICALS S.A) 27 July 2020 (2020-07-27) claims, examples | 1-8 |
| Y | WO 2019/188867 A1 (NOF CORPORATON) 03 October 2019 (2019-10-03) claims, examples | 1-8 |

☐ Further documents are listed in the continuation of Box C.
☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **23 April 2024** | **21 May 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**
**Information on patent family members**

International application No.
**PCT/JP2024/010857**

| Patent document cited in search report | Publication date (day/month/year) | Patent family member(s) | Publication date (day/month/year) |
|---|---|---|---|
| WO 2022/101470 A1 | 19 May 2022 | JP 2023-549266 A<br>US 2023/0414747 A1<br>EP 4243788 A1<br>CN 116829134 A<br>KR 10-2023-0121752 A | |
| WO 2022/060871 A1 | 24 March 2022 | JP 2023-549011 A<br>US 2024/0010609 A1<br>EP 4213882 A1<br>CN 116916904 A | |
| JP 2020-521781 A | 27 July 2020 | US 2021/0128474 A1<br>claims, examples<br>WO 2018/220553 A1<br>EP 3630076 A1<br>CN 110891553 A | |
| WO 2019/188867 A1 | 03 October 2019 | US 2021/0023008 A1<br>claims, examples<br>EP 3778572 A1<br>CN 111902397 A<br>KR 10-2020-0136441 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2008005801 A
- JP 2001002565 A
- WO 2013073480 A
- WO 2019188867 A
- WO 2021195529 A2
- WO 2019188867 A1
- US 20210023008 A1
- US 9708628 B2
- WO 2023053865 A


### Non-patent literature cited in the description


- *Gene Therapy*, 1999, vol. 6, 271-281